(19) Europäisches Patentamt
European Patent Office
Office européen des brevets

(11) Veröffentlichungsnummer: 0 592 980 A2

# (12) EUROPÄISCHE PATENTANMELDUNG

(21) Anmeldenummer: 93116410.7

(22) Anmeldetag: 11.10.93

(51) Int. Cl.5: C09B 62/018

(30) Priorität: 16.10.92 DE 4234900

(43) Veröffentlichungstag der Anmeldung: 20.04.94 Patentblatt 94/16

(84) Benannte Vertragsstaaten: BE CH DE FR GB IT LI

(71) Anmelder: BASF Aktiengesellschaft
Carl-Bosch-Strasse 38
D-67063 Ludwigshafen(DE)

(72) Erfinder: Marschner, Claus, Dr.
Am Wasserturm 7
D-6720 Speyer(DE)
Erfinder: Patsch, Manfred, Dr.
Fritz-Wendel-Strasse 4
D-6706 Wachenheim(DE)

## (54) Reaktivfarbstoffe auf Formazanbasis, die mindestens zwei reaktive Gruppen aufweisen, sowie Aminophenole.

(57) Formazanfarbstoffe der Formel

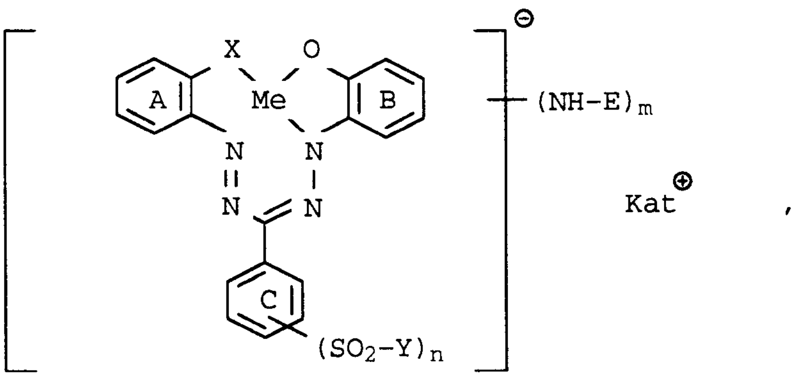

in der

| | |
|---|---|
| m und n | 1 oder 2, |
| Kat$^{\oplus}$ | das Äquivalent eines Kations, |
| Me | Kupfer oder Nickel, |
| X | Sauerstoff oder einen Rest der Formel CO-O oder $SO_2$-O, |
| Y | Vinyl oder einen Rest der Formel $C_2H_4$-Q, worin Q für eine unter alkalischen Reaktionsbedingungen abspaltbare Gruppe steht, und |
| E | einen Ankerrest bedeuten und |

die Ringe A und B gegebenenfalls substituiert sind und benzoanelliert sein können und
der Ring C gegebenenfalls substituiert ist,
deren Verwendung zum Färben oder Bedrucken von Hydroxygruppen oder Stickstoffatome aufweisenden organischen Substraten, neue Aminophenole sowie ein Verfahren zu deren Herstellung.

EP 0 592 980 A2

Die vorliegende Erfindung betrifft neue Formazanfarbstoffe der Formel I

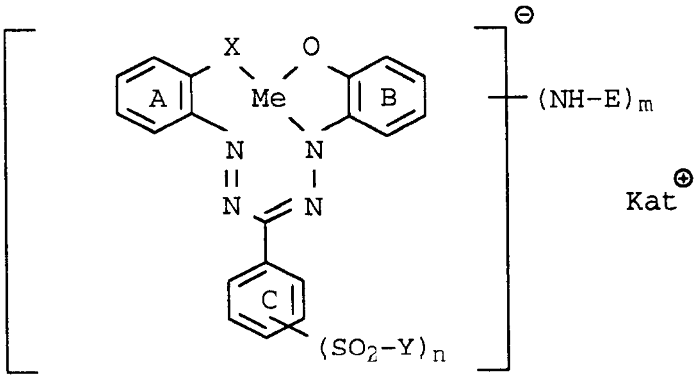

(I),

in der

| | |
|---|---|
| m und n | unabhängig voneinander jeweils 1 oder 2, |
| Kat$^{\oplus}$ | das Äquivalent eines Kations, |
| Me | Kupfer oder Nickel, |
| X | Sauerstoff oder einen Rest der Formel CO-O oder $SO_2$-O, |
| Y | Vinyl oder einen Rest der Formel $C_2H_4$-Q, worin Q für eine unter alkalischen Reaktionsbedingungen abspaltbare Gruppe steht, und |
| E | einen heterocyclischen Ankerrest oder einen Ankerrest der Formel L-W, worin L für ein Brückenglied und W für einen Ankerrest aus der aliphatischen Reihe stehen, bedeuten und |

die Ringe A und B gegebenenfalls substituiert sind und benzoanelliert sein können und der Ring C gegebenenfalls substituiert ist, deren Verwendung zum Färben oder Bedrucken von Hydroxygruppen oder Stickstoffatome aufweisenden organischen Substraten, neue Aminophenole sowie ein Verfahren zu ihrer Herstellung.

Metallkomplex-Formazanfarbstoffe, die reaktive Gruppen aufweisen, sind an sich bekannt und z.B. in der EP-A-28 788 beschrieben. Die Farbstoffe des Standes der Technik weisen jedoch häufig Mängel in ihren anwendungstechnischen Eigenschaften auf.

Aufgabe der vorliegenden Erfindung war es daher, neue Reaktivfarbstoffe auf Formazanbasis bereit zustellen, die sich durch ein vorteilhaftes anwendungstechnisches Eigenschaftsprofil auszeichnen.

Demgemäß wurden die eingangs näher bezeichneten Formazanfarbstoffe der Formel I gefunden.

Hervorzuheben sind Formazanfarbstoffe der Formel Ia

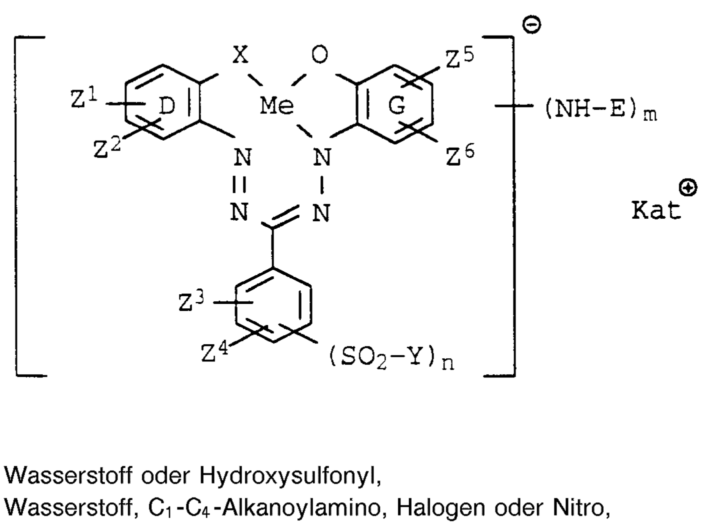

(Ia),

in der

| | |
|---|---|
| $Z^1$ | Wasserstoff oder Hydroxysulfonyl, |
| $Z^2$ | Wasserstoff, $C_1$-$C_4$-Alkanoylamino, Halogen oder Nitro, |
| $Z^3$ und $Z^4$ | unabhängig voneinander jeweils Wasserstoff, Halogen, $C_1$-$C_4$-Alkyl, $C_1$-$C_4$-Alkoxy, $C_1$-$C_4$-Alkylsulfonyl, Nitro oder Hydroxysulfonyl und |
| $Z^5$ und $Z^6$ | unabhängig voneinander jeweils Wasserstoff, Hydroxysulfonyl, Halogen, Nitro, Carboxyl, Ureido, gegebenenfalls substituiertes $C_1$-$C_4$-Mono- oder Dialkylureido, gegebenenfalls |

substituiertes Phenylureido, gegebenenfalls substituiertes Phenyl, gegebenenfalls substituiertes $C_1$-$C_4$-Alkanoylamino, gegebenenfalls substituiertes Benzoylamino, $C_1$-$C_4$-Alkoxycarbonylamino, gegebenenfalls substituiertes Mono- oder Dialkylcarbamoyl oder gegebenenfalls substituiertes Mono- oder Dialkylsulfamoyl bedeuten,

die Ringe D und G benzoanelliert sein können und m, n, Kat$^{\oplus}$ , Me, E, X und Y jeweils die obengenannte Bedeutung besitzen.

Alle in den obengenannten Formeln auftretenden Alkylgruppen können sowohl geradkettig als auch verzweigt sein.

Wenn in den obengenannten Formeln substituierte Alkylgruppen auftreten, so können, sofern nicht anders vermerkt, als Substituenten z.B. Hydroxysulfonyl, Carboxyl, Hydroxy oder Sulfato in Betracht kommen. Die Alkylgruppen weisen dann in der Regel 1 oder 2 Substituenten auf.

Wenn in der obengenannten Formeln substituierte Phenylgruppen auftreten, so können, sofern nicht anders vermerkt, als Substituenten z.B. $C_1$-$C_4$-Alkyl, $C_1$-$C_4$-Alkoxy, Halogen, Nitro, $C_1$-$C_4$-Alkanoyl, $C_1$-$C_4$-Alkanoylamino, Benzoylamino oder Hydroxysulfonyl in Betracht kommen. Die Phenylgruppen weisen dann im allgemeinen 1 bis 3 Substituenten auf.

Kat$^{\oplus}$ stellt des Äquivalent eines Kations dar. Es stellt entweder ein Proton dar oder leitet sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl- Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch Sauerstoffatome in Etherfunktion unterbrochen sein kann.

Besonders als Kationen hervorzuheben sind Protonen oder Lithium-, Natrium- oder Kaliumionen.

Der Rest Q steht für eine unter alkalischen Reaktionsbedingungen abspaltbare Gruppe. Solche Gruppen sind z.B. Chlor, Brom, $C_1$-$C_4$-Alkylsulfonyl, Phenylsulfonyl, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino oder ein Rest der Formel

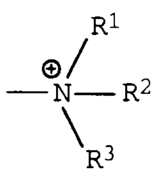

An$^{\ominus}$,

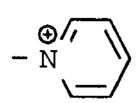

An$^{\ominus}$,

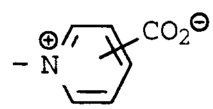

oder

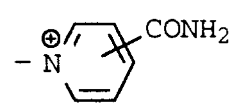

An$^{\ominus}$,

wobei $R^1$, $R^2$ und $R^3$ gleich oder verschieden sind und unabhängig voneinander jeweils die Bedeutung von $C_1$-$C_4$-Alkyl oder Benzyl und An$^{\ominus}$ jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Reste $Z^2$, $Z^3$, $Z^4$, $Z^5$ und $Z^6$ sind z.B. Fluor, Chlor oder Brom.

Reste $Z^5$ und $Z^6$ sind weiterhin z.B. N-Methylureido, N-Ethylureido, N-Propylureido, N-Isopropylureido, N-Butylureido, N-(2-Hydroxysulfonylethyl)ureido, N-(2-Sulfatoethyl)ureido, N,N-Dimethylureido, N-Methyl-N-(2-sulfatoethyl)ureido, Phenylureido, Phenyl, 2-, 3- oder 4-Methylphenyl, 2-, 3- oder 4-Methoxyphenyl, 2-, 3- oder 4-Chlorphenyl, 2-, 3- oder 4-Hydroxysulfonylphenyl, Formylamino, Acetylamino, Propionylamino, Butyrylamino, Isobutyrylamino, 3-Hydroxysulfonylpropionylamino, 3-Carboxypropionylamino, Benzoylamino, 2-, 3- oder 4-Hydroxysulfonylbenzoylamino, 2-, 3- oder 4-Chlorbenzoylamino, 2-, 3- oder 4-Methylbenzoylamino, 2-, 3- oder 4-Carboxylbenzoylamino, Methoxycarbonylamino, Ethoxycarbonylamino, Propoxycarbonylamino, Isopropoxycarbonylamino, Butoxycarbonylamino, Carbamoyl, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, N-Methyl-N-ethylcarbamoyl, N-Methyl-N-(2-hydroxysulfonylethyl)carbamoyl, Sulfamoyl, Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, N-Methyl-N-ethylsulfamoyl oder N-Methyl-N-(2-hydroxysulfonylethyl)sulfamoyl.

Reste $Z^2$ sind weiterhin z.B. Formylamino, Acetylamino, Propionylamino, Butyrylamino oder Isobutyrylamino.

Reste $Z^3$ und $Z^4$ sind weiterhin z.B. Methyl, Ethyl, Propyl, Isopropyl, Butyl, Isobutyl, sec-Butyl, Methoxy, Ethoxy, Propoxy, Isopropoxy, Butoxy, Isobutoxy, sec-Butoxy, Methylsulfonyl, Ethylsulfonyl, Propylsulfonyl, Isopropylsulfonyl oder Butylsulfonyl.

Ankerreste E sind solche, die mit den Hydroxygruppen oder Stickstoffatome enthaltenden Gruppen der zu behandelnden Substrate substitutiv oder additiv reagieren.

Daß der Ankerrest mit den betreffenden Gruppen in den Substraten, z.B. mit den Hydroxygruppen der Cellulose, substitutiv reagiert, bedeutet, daß die Austrittsgruppen oder -atome (z.B. Fluor oder Chlor) im Ankerrest E durch die Hydroxygruppen der Cellulose gemäß folgendem Schema substituiert werden:

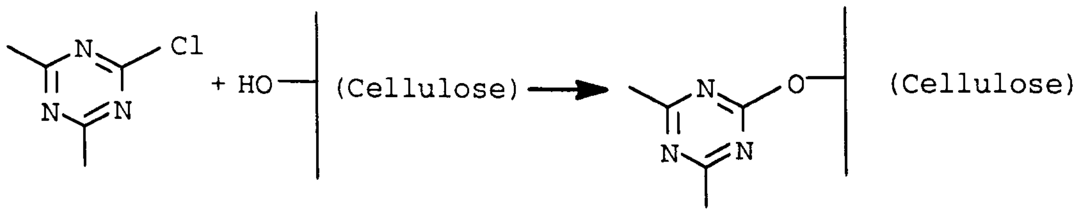

Daß der Ankerrest mit betreffenden Gruppen in den Substraten, z.B. mit Hydroxygruppen der Cellulose, additiv reagiert, bedeutet, daß die Hydroxygruppen der Cellulose gemäß folgendem Schema an den Ankerrest addiert werden:

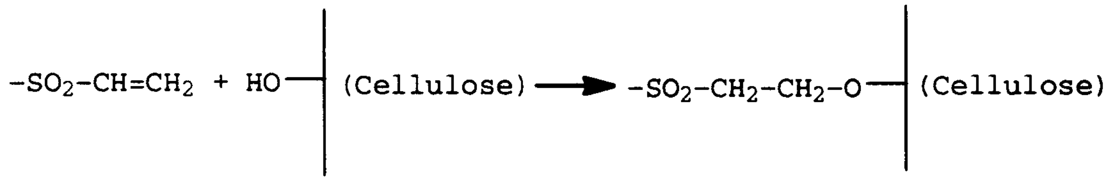

Heterocyclische Ankerreste E sind z.B. halogensubstituierte Reste von 1,3,5-Triazin, Chinoxalin, Phthalazin, Pyrimidin oder Pyridazon, oder der 2-Alkylsulfonylbenzthiazolrest.

Beispielhaft seien folgende heterocyclische Reste genannt:

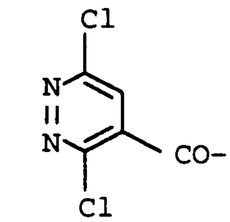 , 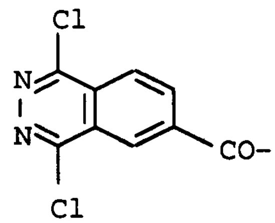 , 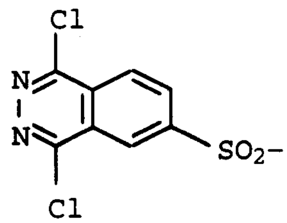 ,

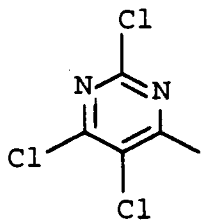 , 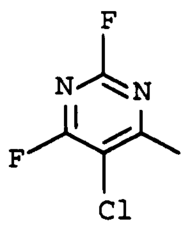 , 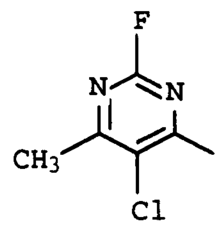 ,

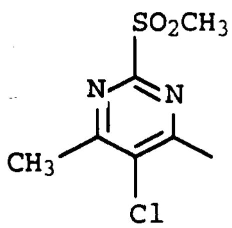 , 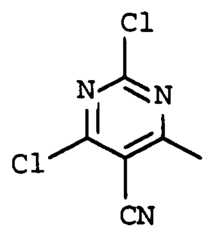 , 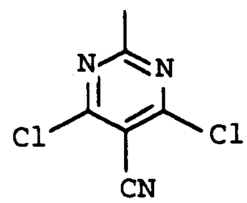 ,

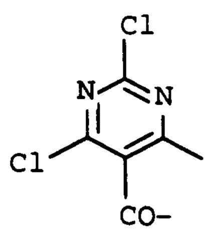 , 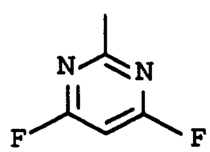 , 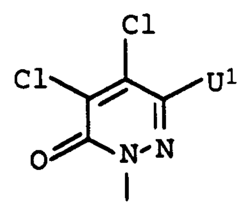 ,

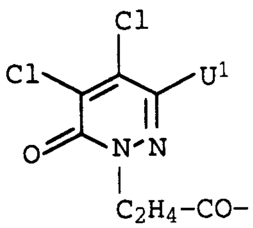 , 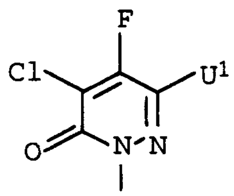 , 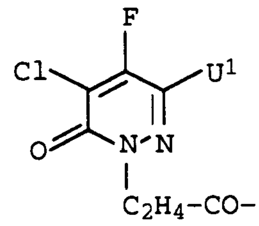 ,

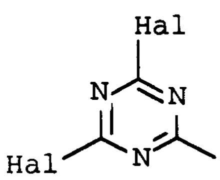 , 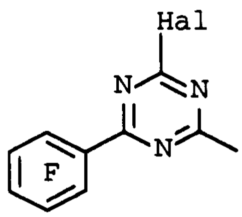 , 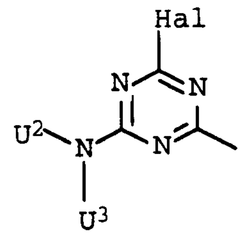 ,

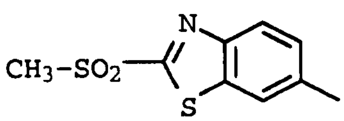 oder 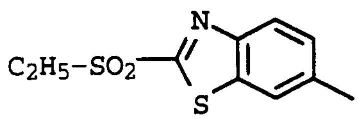 ,

worin

Hal Fluor oder Chlor,

$U^1$ Wasserstoff oder Nitro und

$U^2$ und $U^3$ unabhängig voneinander jeweils Wasserstoff, $C_1$-$C_6$-Alkyl, das gegebenenfalls durch Hydroxy, Halogen, Cyano, Hydroxysulfonyl oder einen Rest der Formel -$SO_2$-Y, worin Y die obengenannte Bedeutung besitzt, substituiert ist und jeweils durch 1 oder 2 Sauerstoffatome in Etherfunktion, Imino- oder $C_1$-$C_4$-Alkyliminogruppen unterbrochen sein kann, oder $U^2$ und $U^3$ zusammen mit dem sie verbindenden Stickstoffatom, Pyrrolidinyl, Piperidinyl, Morpholinyl, Piperazinyl oder N-($C_1$-$C_4$-Alkyl)piperazinyl oder $U^2$ auch einen Rest der Formel

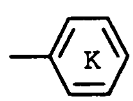 bedeuten,

wobei die Ringe F und K jeweils ein- oder zweifach durch Hydroxysulfonyl substituiert und benzoanelliert sein können und der Ring K davon unabhängig ein- oder zweifach durch Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel $CH_2$-$SO_2$-Y, $SO_2$-Y, NH-CO-Y oder $NU^2$-CO-$NU^2$-$L^1$-$SO_2$-Y, worin Y und $U^2$ jeweils die obengenannte Bedeutung besitzen und $L^1$ für $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 oder 2 Sauerstoffatome in Etherfunktion oder Imino- oder $C_1$-$C_4$-Alkyliminogruppen unterbrochen sein kann, steht, substituiert sein kann.

Ankerreste W aus der aliphatischen Reihe sind beispielsweise Acryloyl-, Mono-, Di- oder Trichloracryloyl, -CO-CCl=CH-COOH, -CO-CH=CCl-COOH, 2-Chlorpropionyl, 3-Phenylsulfonylpropionyl, 3-Methylsulfonylpropionyl, 2-Sulfatoethylaminosulfonyl, 2-Fluor-2-chlor-3,3-difluorcyclobut-1-ylcarbonyl, 2,2,3,3-Tetrafluorcyclobut-1-ylcarbonyl, 2,2,3,3-Tetrafluorcyclobut-1-ylsulfonyl, 2-(2,2,3,3-Tetrafluorcyclobut-1-yl)acryloyl, 1- oder 2-Bromacryloyl, 1- oder 2-Alkyl- oder -Arylsulfonylacryloyl, wie 1- oder 2-Methylsulfonylacryloyl, 1,2-Dichlorpropionyl, 1,2-Dibrompropionyl oder ein Rest der Formel $SO_2$-Y, worin Y die obengenannte Bedeutung besitzt.

Geeignete Brückenglieder L, die den aliphatischen Ankerrest W mit der Aminogruppe des Formazans verknüpfen, gehorchen z.B. der Formel

$-CO-L^1-$ , 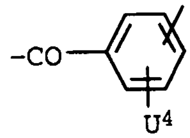 , 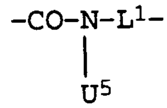 ,

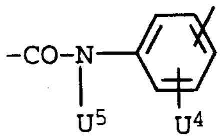 oder 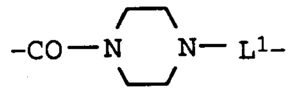 ,

worin

$U^4$ Wasserstoff, $C_1$-$C_4$-Alkyl, Carboxyl, $C_1$-$C_4$-Alkoxy, Halogen, Nitro oder Hydroxysulfonyl und

$U^5$ Wasserstoff, $C_1$-$C_6$-Alkyl, das gegebenenfalls durch Hydroxy, $C_1$-$C_4$-Alkoxy, Halogen, Cyano, Hydroxysulfonyl oder einen Rest der Formel $-SO_2-Y$, wobei Y die obengenannte Bedeutung besitzt, substituiert ist, oder gegebenenfalls substituiertes Phenyl bedeuten und $L^1$ die obengenannte Bedeutung besitzt.

Der Ankerrest E kann auch einen weiteren Formazanrest aufweisen, der entweder mit dem bereits vorhandenen Formazanrest identisch oder verschieden davon ist.

Besonders zu nennen in diesem Falle sind Kupferformazanfarbstoffe der Formel V

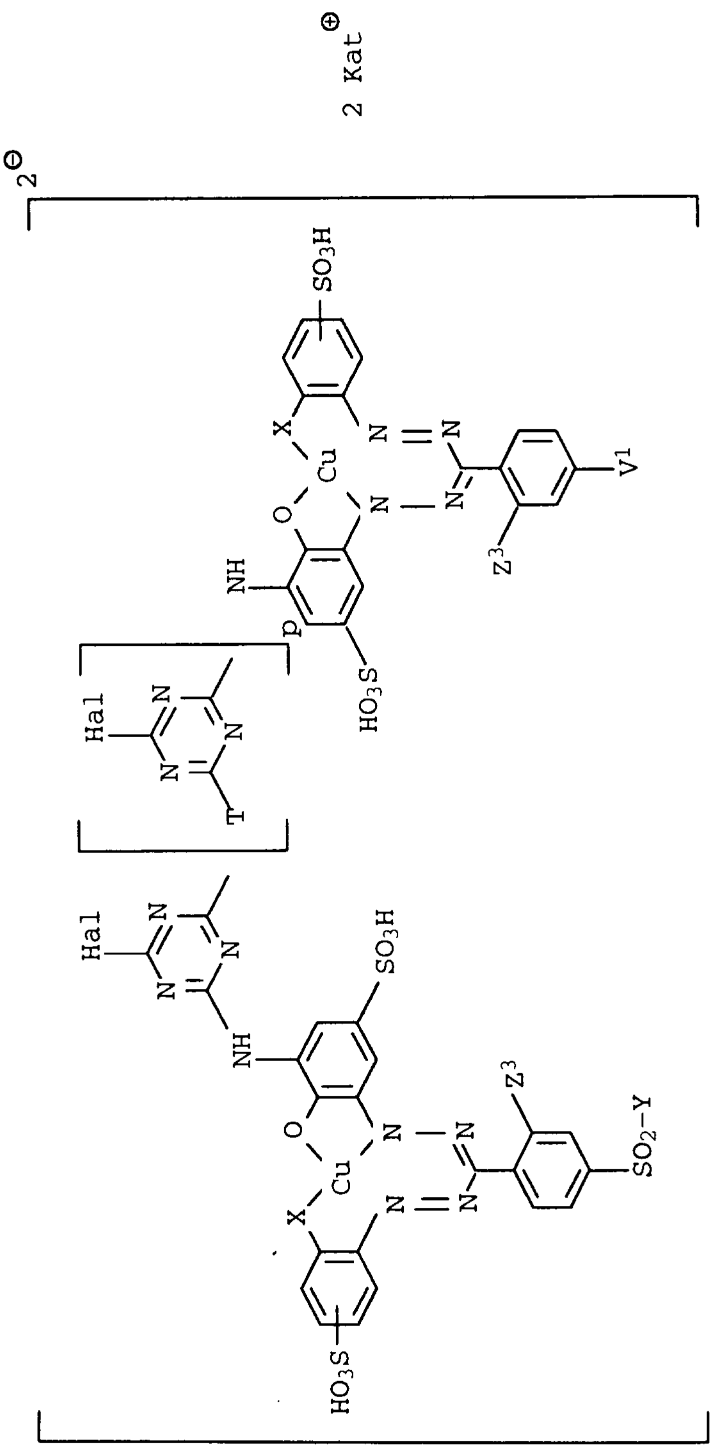

(V),

in der

p 0 oder 1,

T einen Rest der Formel

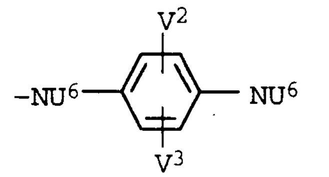

,

$NU^6$-$L^1$-$NU^6$ oder

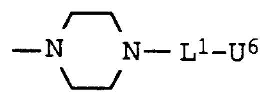

,

worin $U^6$ für Wasserstoff oder $C_1$-$C_4$-Alkyl und $V^2$ und $V^3$ unabhängig voneinander jeweils für Wasserstoff oder Hydroxysulfonyl stehen und $L^1$ jeweils die obengenannte Bedeutung besitzt, und

$V^1$ Wasserstoff oder den Rest $SO_2$-Y bedeuten und

Hal, X, Y, $Z^3$ und $Kat^{\oplus}$ jeweils die obengenannte Bedeutung besitzen.

Bevorzugt sind Formazanfarbstoffe der Formel I, in der Me Kupfer bedeutet.

Weiterhin bevorzugt sind Formazanfarbstoffe der Formel I, in der m und n jeweils 1 bedeuten.

Weiterhin bevorzugt sind Formazanfarbstoffe der Formel I, in der Y Vinyl oder einen Rest der Formel $C_2H_4$-Q bedeutet, worin Q für Chlor, Sulfato oder Thiosulfato steht, wobei Chlor besonders hervorzuheben ist.

Weiterhin bevorzugt sind Formazanfarbstoffe der Formel I, in der X den Rest der Formel CO-O bedeutet.

Besonders bevorzugt sind Formazanfarbstoffe, die der Formel Ib

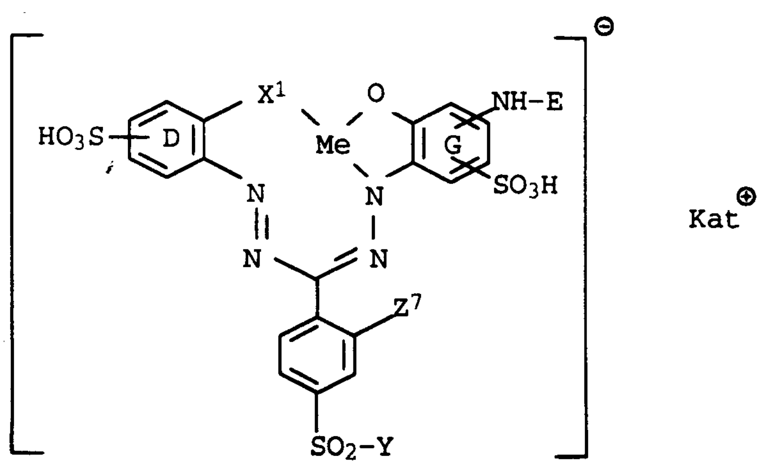

(Ib)

entsprechen, in der

$Z^7$ Wasserstoff, $C_1$-$C_4$-Alkyl, Hydroxysulfonyl oder Halogen und

$X^1$ einen Rest der Formel CO-O oder $SO_2$-O bedeuten und

Me, Y, E, $Kat^{\oplus}$ und die Ringe D und G jeweils die obengenannte Bedeutung besitzen.

Hervorzuheben sind Formazanfarbstoffe der Formel Ic

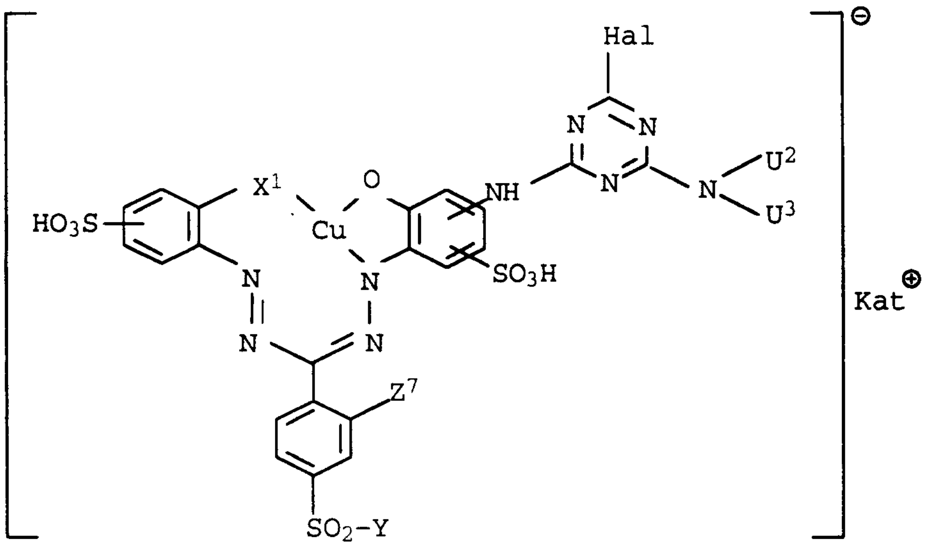

Kat$^{\oplus}$ (Ic),

in der $U^2$, $U^3$, Hal, $X^1$, Y, $Z^7$ und Kat$^{\oplus}$ jeweils die obengenannte Bedeutung besitzen.

Hervorzuheben sind weiterhin Formazanfarbstoffe der Formel Id

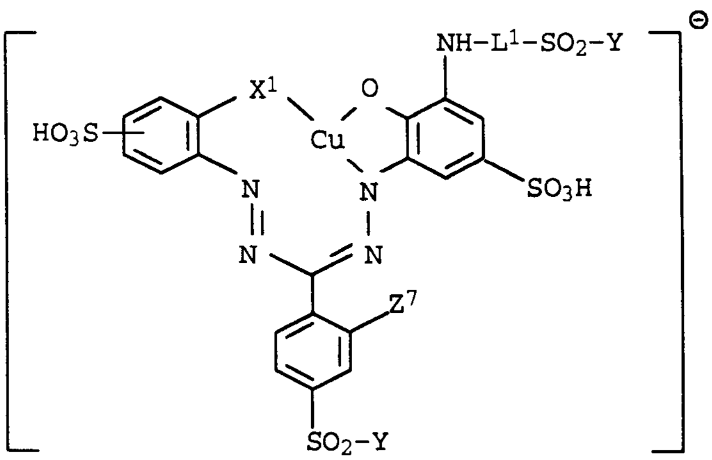

Kat$^{\oplus}$ (Id),

in der $L^1$, $X^1$, Y, $Z^7$ und Kat$^{\oplus}$ jeweils die obengenannte Bedeutung besitzen.

Von besonderem Interesse sind Formazanfarbstoffe der Formel Ie

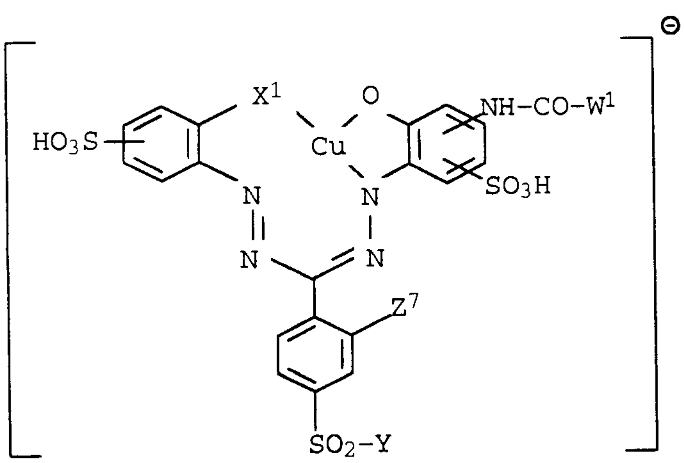

Kat$^{\oplus}$ (Ie),

in der

W¹ einen Rest der Formel $alk^1$-$SO_2$-Y oder

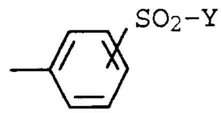

,

worin $alk^1$ für $C_3$-$C_5$-Alkylen steht, bedeutet und

$X^1$, Y, $Z^7$ und $Kat^{\oplus}$ jeweils die obengenannte Bedeutung besitzen.

Von besonderem Interesse sind weiterhin Formazanfarbstoffe der Formel If

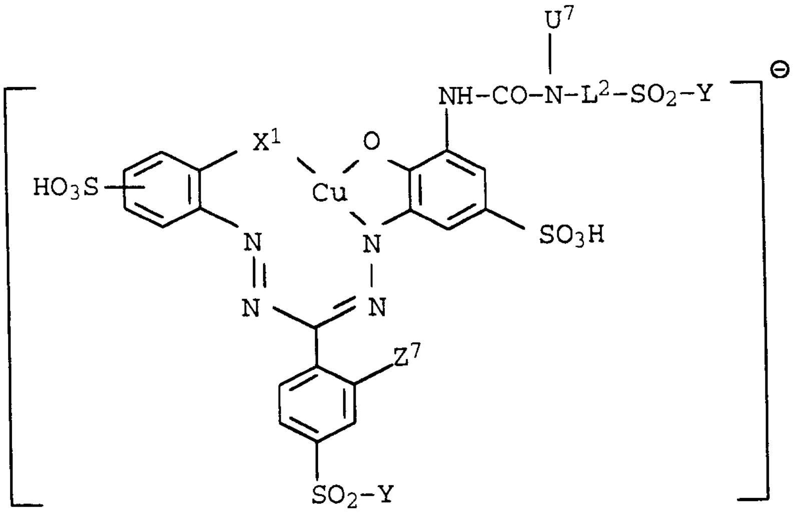

Kat$^{\oplus}$ (If),

in der

$U^7$ Wasserstoff oder $C_1$-$C_4$-Alkyl, das gegebenenfalls durch einen Rest der Formel $SO_2$-Y substituiert ist und durch ein Sauerstoff in Etherfunktion unterbrochen sein kann, und

$L^2$ einen Rest der Formel

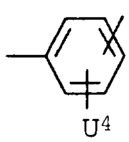

,

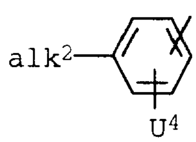

,

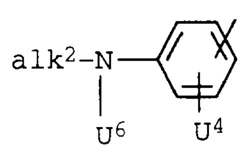

oder

-$alk^2$- bedeuten, worin $U^4$ und $U^6$ jeweils die obengenannte Bedeutung besitzen und $alk^2$ jeweils für $C_2$-$C_4$-Alkylen, das gegebenenfalls durch ein Sauerstoffatom in Etherfunktion unterbrochen ist, steht und

$X^1$, Y, $Z^7$ und $Kat^{\oplus}$ jeweils die obengenannte Bedeutung besitzen.

Die vorliegende Erfindung betrifft weiterhin Aminophenole der Formel II

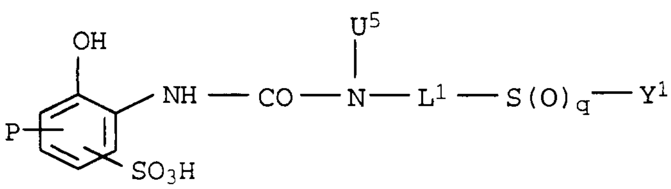

(II),

in der

q 0 oder 2,

$Y^1$ Vinyl oder einen Rest der Formel $C_2H_4$-$Q^1$, worin $Q^1$ für eine unter alkalischen Reaktionsbedingungen abspaltbare Gruppe oder Hydroxy steht,

- $L^1$ $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 bis 2 Sauerstoffatome in Etherfunktion oder Imino- oder $C_1$-$C_4$-Alkyliminogruppen unterbrochen sein kann,
- $U^5$ Wasserstoff, $C_1$-$C_6$-Alkyl, das gegebenenfalls durch Hydroxy, $C_1$-$C_4$-Alkoxy, Halogen, Cyano, Hydroxysulfonyl oder einen Rest der Formel -$SO_2$-$Y^1$, wobei $Y^1$ die obengenannte Bedeutung besitzt, substituiert ist, oder gegebenenfalls substituiertes Phenyl und
- P Nitro oder Amino bedeuten.

Bevorzugt sind Aminophenole der Formel IIa

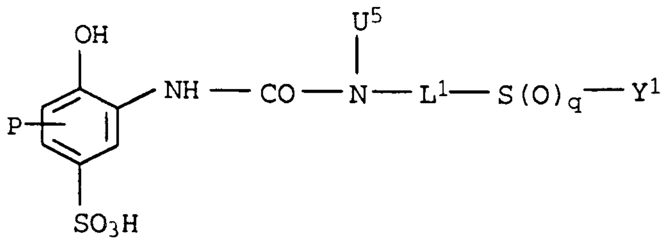

(IIa),

in der q, $Y^1$, $L^1$, $U^5$ und P jeweils die obengenannte Bedeutung besitzen.

Besonders zu nennen sind Aminophenole der Formel II, in der $L^1$ $C_2$-$C_4$-Alkylen, das gegebenenfalls durch ein Sauerstoffatom in Etherfunktion unterbrochen ist, bedeutet.

Besonders zu nennen sind weiterhin Aminophenole der Formel II, in der $U^5$ Wasserstoff oder $C_1$-$C_4$-Alkyl, das gegebenenfalls durch einen Rest der Formel $SO_2$-$Y^1$ substituiert ist und durch ein Sauerstoffatom in Etherfunktion unterbrochen sein kann, bedeutet.

Die neuen Aminophenole der Formel II sind wertvolle Zwischenprodukte für die Herstellung der Formazanfarbstoffe der Formel I.

Es wurde weiterhin gefunden, daß man die Aminophenole der Formel II vorteilhaft erhält, wenn man ein Benzoxazolon der Formel III

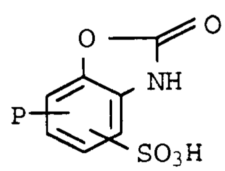

(III),

in der P die obengenannte Bedeutung besitzt, mit einem Amin der Formel IV

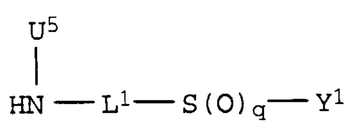

(IV),

in der q, $U^5$, $L^1$ und $Y^1$ jeweils die obengenannte Bedeutung besitzen, umsetzt.

Das erfindungsgemäße Verfahren wird zweckmäßig in Wasser oder in einem polaren organischen Lösungsmittel, z.B. in einem $C_1$-$C_4$-Alkanol oder in N,N-Dimethylformamid, bei einer Temperatur von 50 bis 95 °C und einem pH-Wert von 5 bis 9, bevorzugt 6 bis 7,5, vorgenommen.

Anschließend kann man nach an sich bekannten Methoden gegebenenfalls die Thioether- zur Sulfongruppe oxidieren, die Nitro- zur Aminogruppe reduzieren und die unter alkalischen Bedingungen abspaltbare Gruppe Q einführen.

Die Benzoxazolone der Formel III können nach an sich bekannten Methoden, z.B. durch Umsetzung der entsprechenden o-Aminophenole mit Phosgen, wie in den Beispielen näher erläutert, erhalten werden.

In einer bevorzugten Variante werden die bei der Umsetzung dieser o-Aminophenole mit Phosgen entstehenden Benzoxazolone direkt, d.h. ohne Zwischenisolierung, im erfindungsgemäßen Herstellverfahren verwendet.

Die neuen Formazanfarbstoffe der Formel I können nach an sich bekannten Methoden erhalten werden. Beispielsweise kann man ein Hydrazon der Formel VI

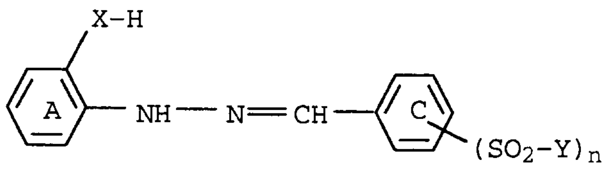

(VI),

in der n, X, Y, und die Ringe A und C jeweils die obengenannte Bedeutung besitzen, mit einem Diazoniumsalz, das sich von einem Formylaminophenol der Formel VII ableitet

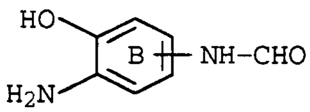

(VII),

in der
der Ring B die obengenannte Bedeutung besitzt, in Gegenwart eines Kupfer- oder Nickelsalzes, z.B. Kupfersulfat oder Nickelsulfat, umsetzen und anschließend gemäß der in der älteren europäischen Patentanmeldung 93 109 173.0 beschriebenen Methode hydrolysieren. Die Hydrazone der Formel VI sind aus der älteren deutschen Patentanmeldung P 42 30 095.9 bekannt. Die Herstellung der Formylaminophenole VII ist in der älteren Patentanmeldung 93 109 173.0 beschrieben.

Der nach der Hydrolyse resultierende Farbstoff der Formel VIII

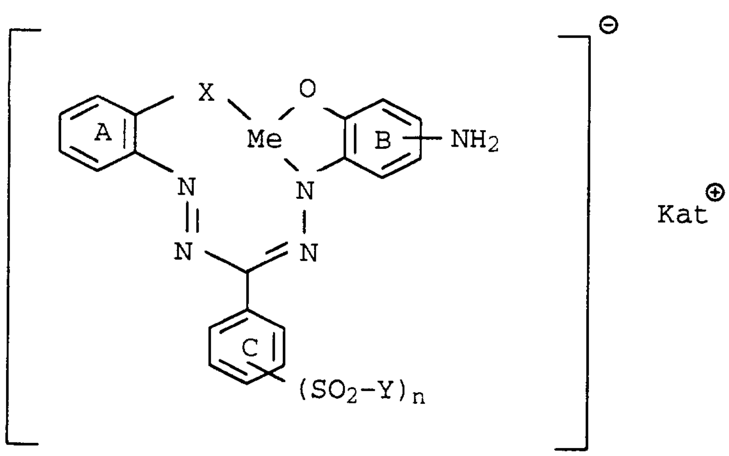

(VIII),

in der n, Me, X, Y, Kat$^{\oplus}$ und die Ringe A, B und C jeweils die obengenannte Bedeutung besitzen, kann dann mit einer Ankerkomponente der Formel IX

E-Lg (IX),

in der Lg eine Austrittsgruppe, z.B. Chlor oder Brom bedeutet, und E die obengenannte Bedeutung besitzt, weiter umgesetzt werden.

Bei der Herstellung von solchen Reaktivfarbstoffen der Formel I, die einen Triazinring aufweisen, stellt die Ankerkomponente IX z.B. Cyanurchlorid dar. Nach der Umsetzung mit dem Farbstoff der Formel VIII erfolgt in diesem Falle noch die Reaktion mit dem Amin der Formel X

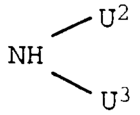

(X),

in der $U^2$ und $U^3$ jeweils die obengenannte Bedeutung besitzen. Es ist auch möglich, Cyanurchlorid zunächst mit dem Amin X zu kondensieren und das resultierende Triazinderivat anschließend mit dem Farbstoff VIII umzusetzen.

Es können aber auch, insbesondere bei der Herstellung der Formazanfarbstoffe der Formel Id, die in der US-A-5 023 274 oder US-A-4 841 028, auf die hier beide ausdrücklich Bezug genommen wird, näher beschriebenen Methoden angewandt werden.

Ein vorteilhaftes Verfahren zur Herstellung der Formazanfarbstoffe der Formel If besteht weiterhin darin, Hydrazone der Formel VI mit einem Diazoniumsalz, das sich von einem Aminophenol der Formel XI

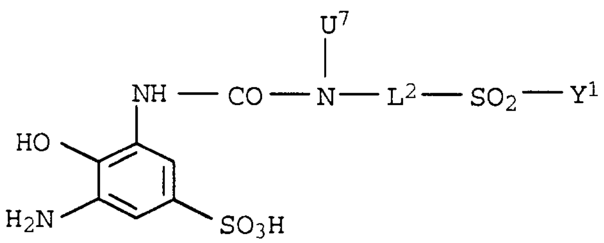

(XI)

ableitet, in der $U^7$, $L^2$ und Y jeweils die obengenannte Bedeutung besitzen, in Gegenwart eines Kupfer- oder Nickelsalzes, z.B. Kupfersulfat oder Nickelsulfat, umzusetzen.

Die neuen Formazanfarbstoffe der Formel I eignen sich in vorteilhafter Weise zum Färben oder Bedrucken von Hydroxygruppen oder Stickstoffatome aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Wolle oder insbesondere von Baumwolle. Man erhält Ausfärbungen in rotstichig blauen Farbtönen.

Insbesondere auf Substraten auf Basis von Cellulose werden farbstarke Färbungen mit sehr hoher Fixierausbeute erhalten, die eine sehr gute Lichechtheit sowie vorzügliche Naßechtheiten, wie Wasch-, Chlorbleich-, Peroxidbleich-, Alkali-, Meerwasser- oder Schweißechtheit, aufweisen.

Die folgenden Beispiele sollen die Erfindung näher erläutern.

Beispiel 1

34,8 g 2-Formylamino-6-aminophenol-4-sulfonsäure wurden in 300 ml Wasser gelöst, auf 0 bis 5 °C abgekühlt und der pH-Wert mit 50 ml konz. Salzsäure auf 2 gestellt. Anschließend wurde bei einer Temperatur von 0 bis 5 °C durch Zugabe von 40 g 23 gew.-%iger wäßriger Natriumnitritlösung diazotiert.

Die resultierende Diazosuspension ließ man bei 10 bis 15 °C zu einer auf einen pH-Wert von 6,5 bis 7,0 gestellten Mischung aus 53,6 g des Hydrazons der Formel

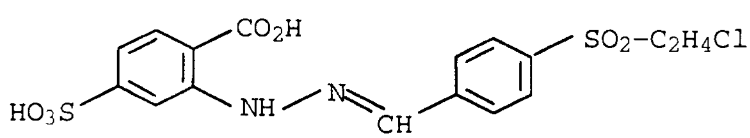

und 34,9 g Kupfersulfat-pentahydrat in 300 ml Wasser laufen, wobei der pH-Wert durch Einstreuen von Natriumhydrogencarbonat zwischen 6,5 und 7,0 gehalten wurde. Nach Beendigung des Zulaufs ließ man noch 12 Stunden bei 15 bis 20 °C nachrühren.

Anschließend wurde auf 60 °C erwärmt, der pH-Wert mit konz. Salzsäure auf 1 gestellt und 3 Stunden bei 60 °C nachgerührt.

Nach vollständiger Verseifung (DC-Kontrolle) wurde der pH-Wert mit Natriumhydrogencarbonat auf 6,5 gestellt, das ausgefallene Produkt abfiltriert und bei 50 ° C unter vermindertem Druck getrocknet.

Man erhielt 95 g eines elektrolythaltigen Kupferformazans der Formel

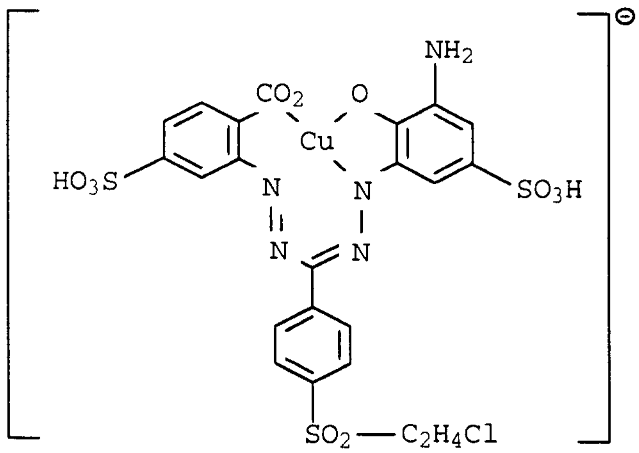

$Na^{\oplus}$

($\lambda_{max}$ = 572 nm)

In analoger Weise werden die in der folgenden Tabelle 1 aufgeführten Formazane erhalten.

Tabelle 1

| Bsp.-Nr. | Kupferformazan | |
|---|---|---|
| 2 | 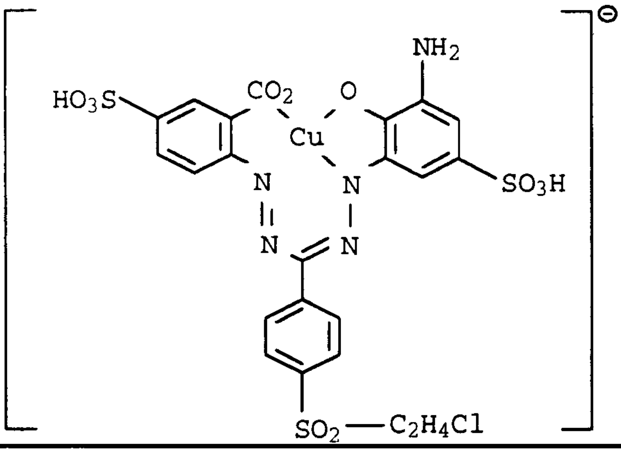 | Na$^{\oplus}$ |
| 3 | 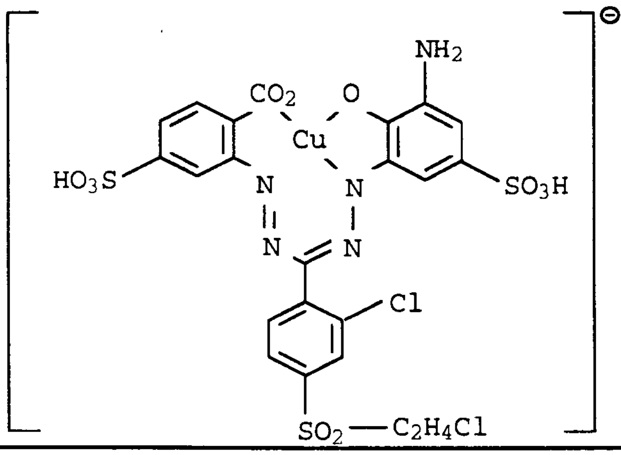 | Na$^{\oplus}$ |
| 4 | 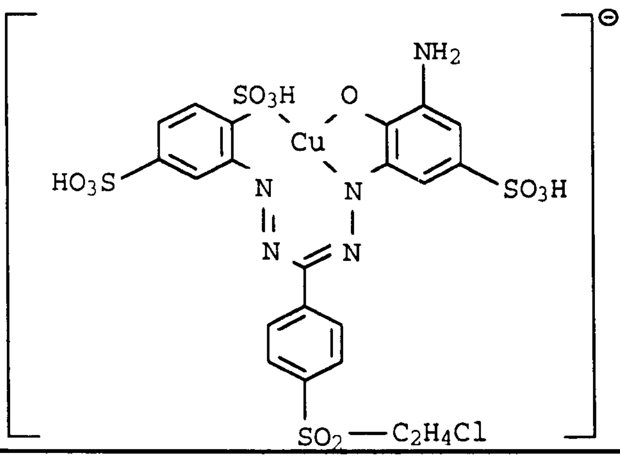 | Na$^{\oplus}$ |

| Bsp.-Nr. | Kupferformazan |
|---|---|
| 5 | 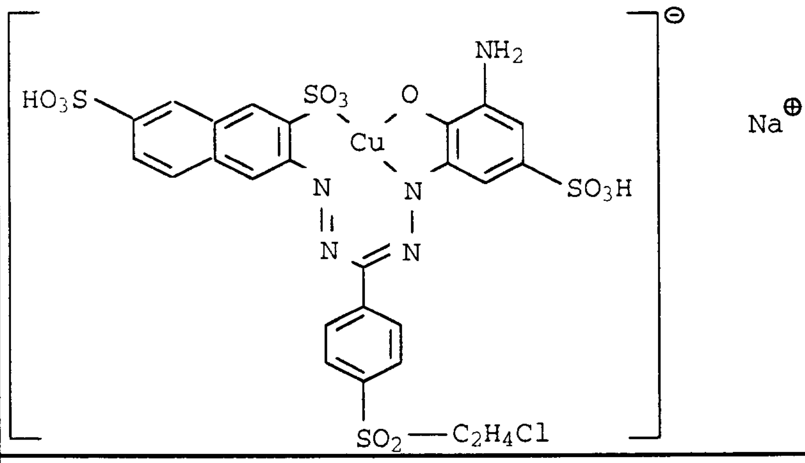 |
| 6 | 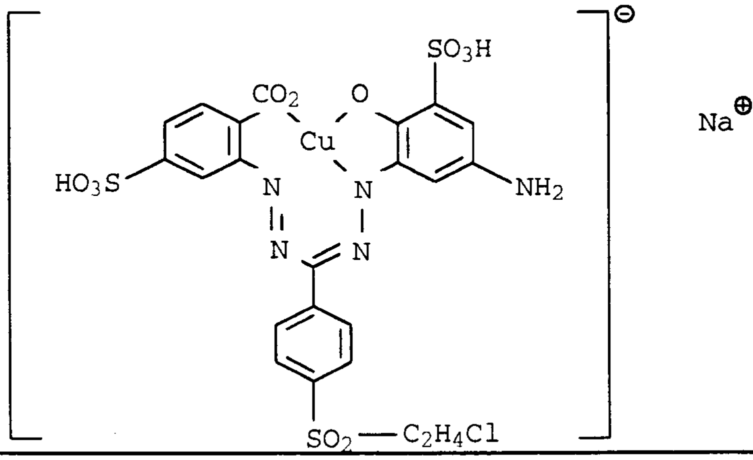 |

Beispiel 7

a) 19 g der Kupferformazanverbindung der Formel

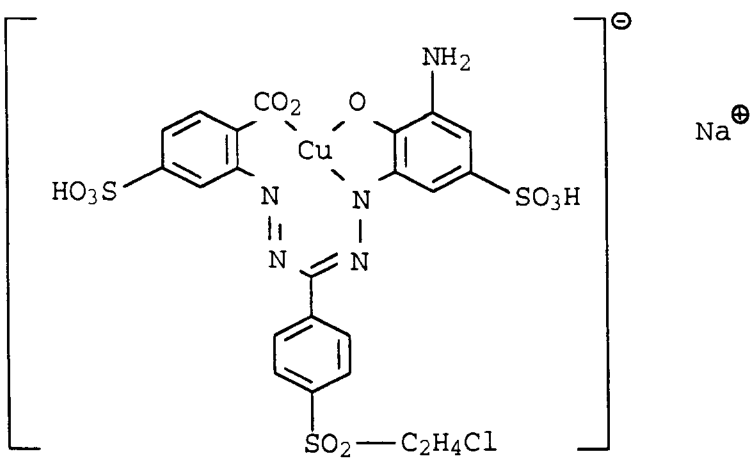

wurden in 200 ml Wasser gelöst. Man setzte 0,6 g Dinatriumhydrogenphosphat zu, kühlte auf 0 bis 5 °C ab und ließ innerhalb von 5 Minuten eine Lösung von 3,69 g Cyanurchlorid in 25 ml Aceton zulaufen. Der pH-Wert wurde mit 5 gew.-%iger wäßriger Natriumhydrogencarbonatlösung bei 6,0 bis 6,5 gehalten und die Kondensation unter obigen Bedingungen zu Ende geführt (DC-Kontrolle), was ca. 1 Stunde in Anspruch nahm.

b) Anschließend wurde eine auf einen pH-wert von 6 eingestellte Lösung von 5 g Anilin-3-sulfonsäure in 200 ml Wasser zugegeben. Die Temperatur wurde auf 60 °C erhöht, dann wurde eine Stunde bei dieser Temperatur nachgerührt, wobei der pH-Wert mit 5 gew.-%iger wäßriger Natriumhydrogencarbonatlösung zwischen 6,0 und 6,5 gehalten wurde.

Der entstandene Reaktivfarbstoff wurde durch Zusatz von 150 g Kochsalz ausgesalzen. Man erhielt einen Farbstoff der Formel

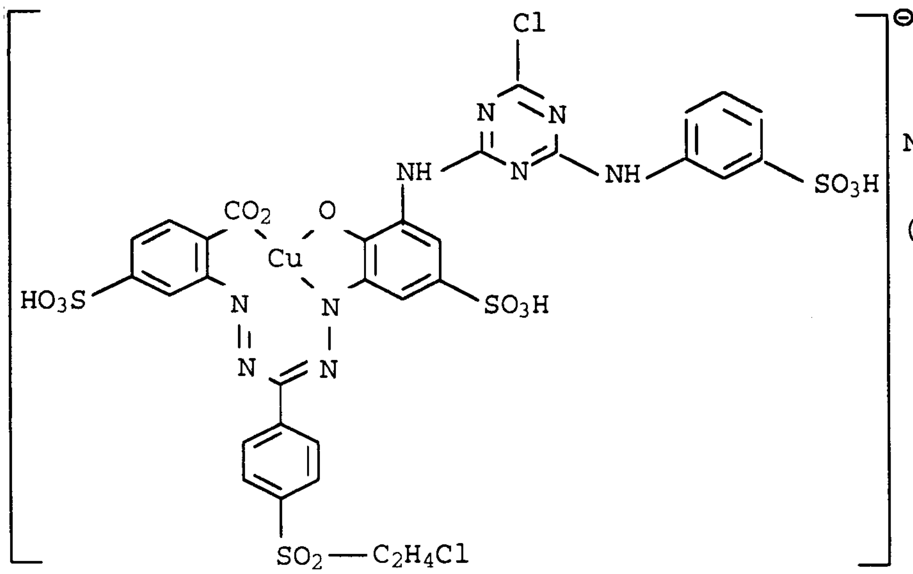

$Na^{\oplus}$

($\lambda_{max}$ = 591 nm)

der Baumwolle in rotstichigem Blauton mit sehr guten Echtheiten färbt.

Beispiel 8

Man verfuhr analog Beispiel 7, verwendete jedoch anstelle von Anilin-3-sulfonsäure 5,13 g Taurin, und erhielt nach dem Aussalzen einen Farbstoff der Formel

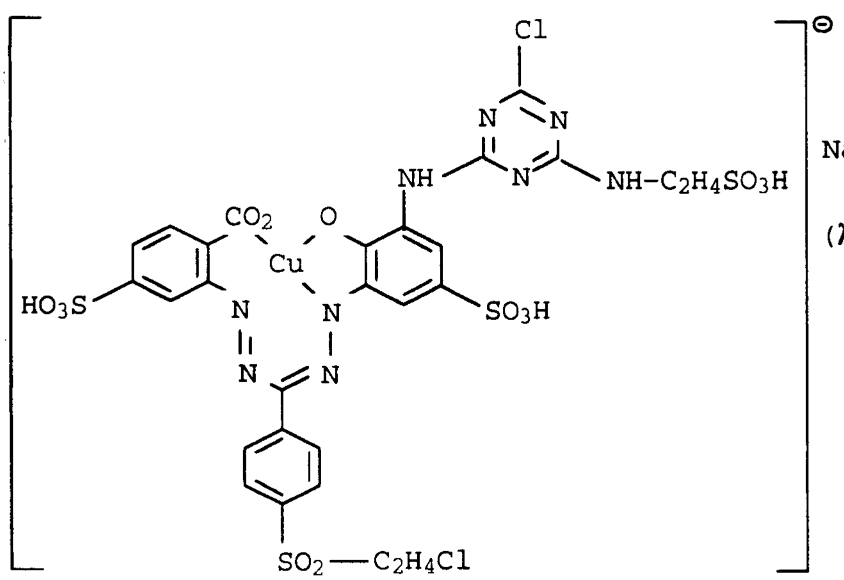

$Na^{\oplus}$

($\lambda_{max}$ = 601 nm)

der Baumwolle in rotstichigem Blauton mit sehr guten Echtheiten färbt.

Beispiel 9

9,33 g 4-(2-Sulfatoethyl)sulfonylanilin wurden in 200 ml Wasser bei einem pH-Wert von 6,5 und 0 bis 5 °C gelöst. Es wurde eine Suspension von 5,53 g Cyanurchlorid in 50 g Eiswasser zugegeben und bei 0 bis 5 °C und einem pH-Wert von 6,5 2 Stunden nachgerührt.

Die so erhaltene Suspension des Dichlortriazins der Formel

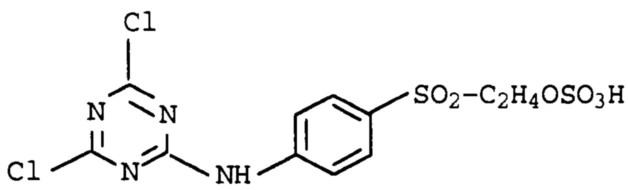

wurde mit einer Lösung von 15,7 g des in Beispiel 1 beschriebenen Kupferformazans in 150 ml Wasser bei 10 bis 15 °C versetzt.

Es wurde 6 Stunden bei 10 bis 15 °C nachgerührt, wobei der pH-Wert mit 5 gew.-%iger wäßriger Natriumhydrogencarbonatlösung bei 6,0 bis 6,5 gehalten wurde.

Der entstandene Reaktivfarbstoff wurde durch Zusatz von 300 g Kochsalz ausgesalzen.

Man erhielt einen Farbstoff der Formel

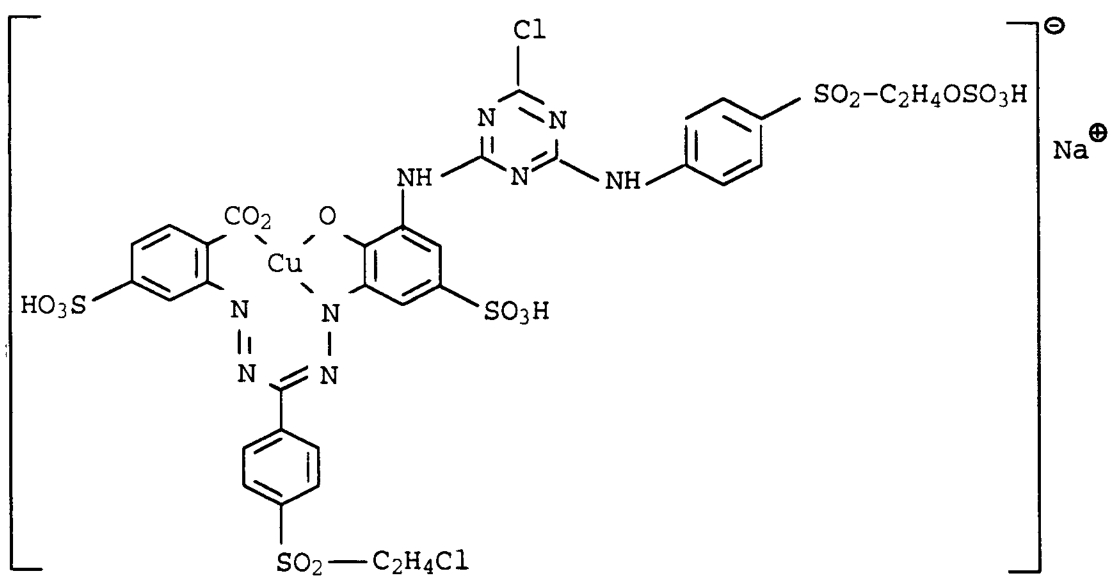

($\lambda_{max}$ = 606 nm)

der Baumwolle in rotstichigem Blauton mit sehr guten Echtheiten färbt.

In analoger Weise werden die in den folgenden Tabellen 2 und 3 aufgeführten Formazanfarbstoffe erhalten.

Tabelle 2

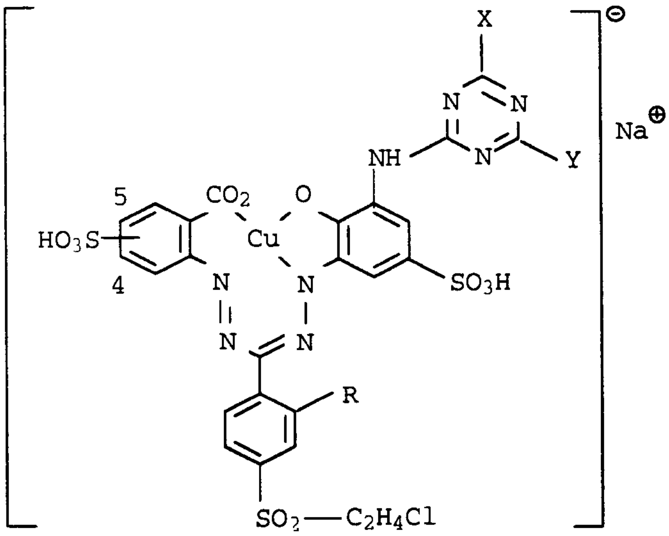

| Bsp.-Nr. | Position von $SO_3H$ | R | X | Y |
|---|---|---|---|---|
| 10 | 4 | H | Cl | 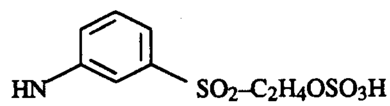 |
| 11 | 4 | H | Cl | 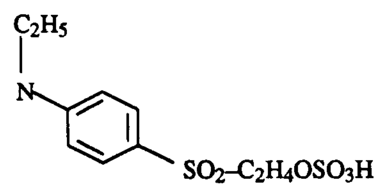 |
| 12 | 4 | H | Cl | 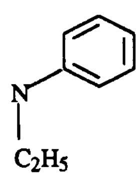 |
| 13 | 4 | H | Cl | 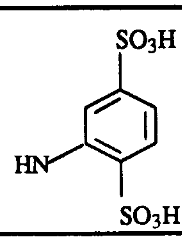 |

| Bsp.-Nr. | Position von $SO_3H$ | R | X | Y |
|---|---|---|---|---|
| 14 | 4 | H | Cl | 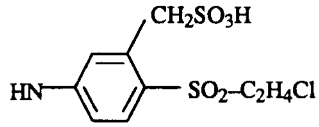 |
| 15 | 4 | H | Cl | 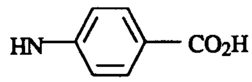 |
| 16 | 4 | H | Cl | 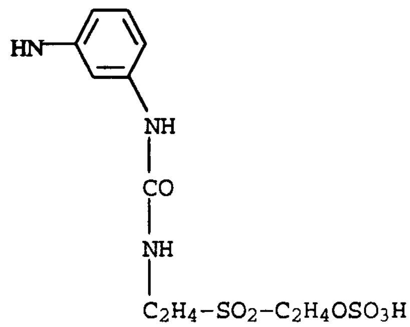 |
| 17 | 4 | H | Cl | 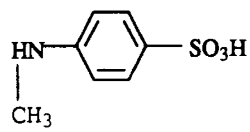 |
| 18 | 4 | H | Cl | 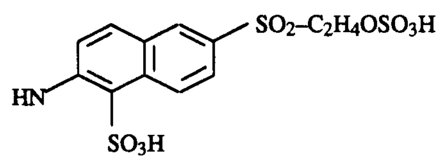 |
| 19 | 4 | H | Cl | 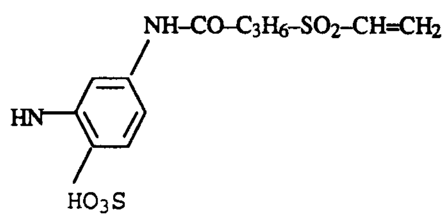 |
| 20 | 4 | H | Cl | 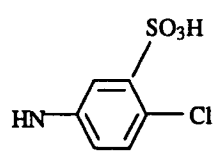 |

| Bsp.-Nr. | Position von $SO_3H$ | R | X | Y |
|---|---|---|---|---|
| 21 | 4 | H | Cl | 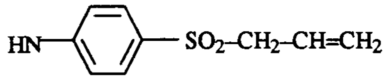 |
| 22 | 4 | H | Cl | $NH_2$ |
| 23 | 4 | H | Cl | $NHCH_3$ |
| 24 | 4 | H | Cl | $NHCH_2CO_2H$ |
| 25 | 4 | H | Cl | $N(CH_3)_2$ |
| 26 | 4 | H | Cl | 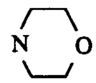 |
| 27 | 4 | H | Cl | $NH-C_2H_4OH$ |
| 28 | 4 | H | Cl | $NH-C_2H_4-SO_2-C_2H_4Cl$ |
| 29 | 4 | H | Cl | $NH-C_2H_4-SO_2-C_2H_4OSO_3H$ |
| 30 | 4 | H | Cl | 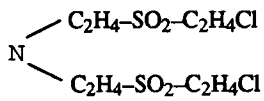 |
| 31 | 4 | H | Cl | $NH-C_2H_4OC_2H_4-SO_2-CH=CH_2$ |
| 32 | 4 | H | Cl | 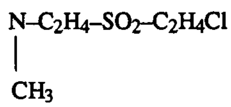 |
| 33 | 4 | H | Cl | $NH-C_3H_6-SO_2-C_2H_4Cl$ |
| 34 | 4 | H | F | 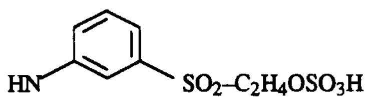 |
| 35 | 5 | H | Cl | 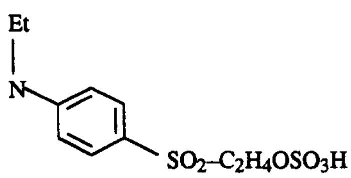 |
| 36 | 5 | H | Cl | 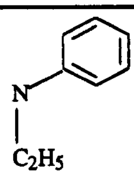 |

| Bsp.-Nr. | Position von $SO_3H$ | R | X | Y |
|---|---|---|---|---|
| 37 | 4 | Cl | Cl | 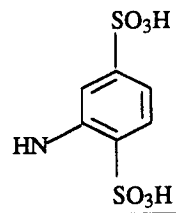 |
| 38 | 4 | H | F | 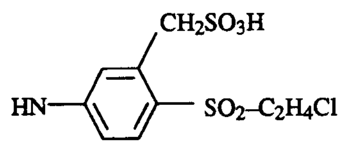 |
| 39 | 4 | Cl | Cl | 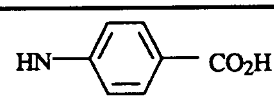 |
| 40 | 4 | H | F | 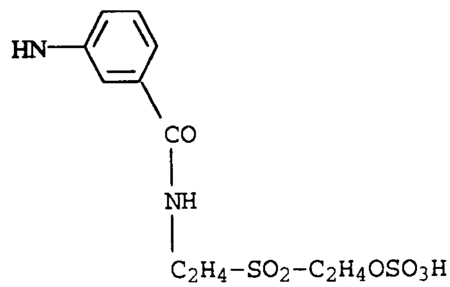 |
| 41 | 5 | H | F | 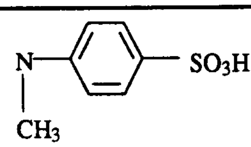 |
| 42 | 5 | H | Cl | 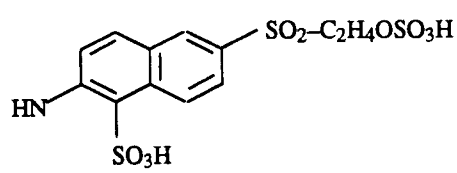 |
| 43 | 5 | H | F | 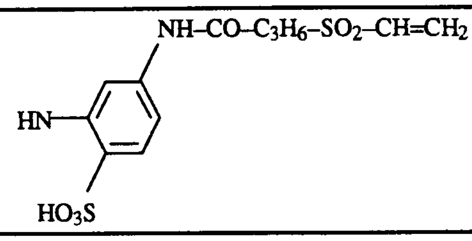 |

| Bsp.-Nr. | Position von $SO_3H$ | R | X | Y |
|---|---|---|---|---|
| 44 | 4 | Cl | Cl | 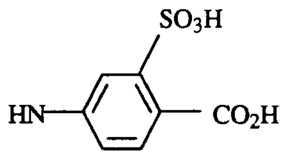 |
| 45 | 4 | H | F | 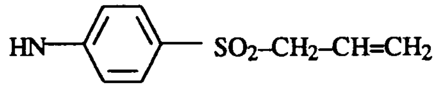 |
| 46 | 5 | H | Cl | $NH_2$ |
| 47 | 5 | Cl | Cl | $NHCH_3$ |
| 48 | 5 | H | Cl | $NHCH_2CO_2H$ |
| 49 | 5 | Cl | Cl | $N(CH_3)_2$ |
| 50 | 4 | Cl | Cl | 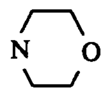 |
| 51 | 4 | Cl | F | $NH-C_2H_4OH$ |
| 52 | 4 | Cl | F | $NH-C_2H_4-SO_2-C_2H_4Cl$ |
| 53 | 4 | H | F | $NH-C_2H_4-SO_2-C_2H_4OSO_3H$ |
| 54 | 4 | H | F | 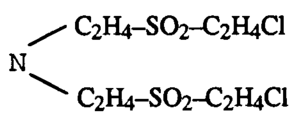 |
| 55 | 5 | H | F | $NH-C_2H_4OC_2H_4-SO_2-CH=CH_2$ |
| 56 | 5 | H | F | 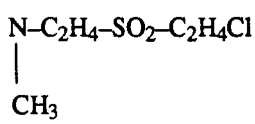 |
| 57 | 5 | H | F | $NH-C_3H_6-SO_2-C_2H_4Cl$ |

Tabelle 3

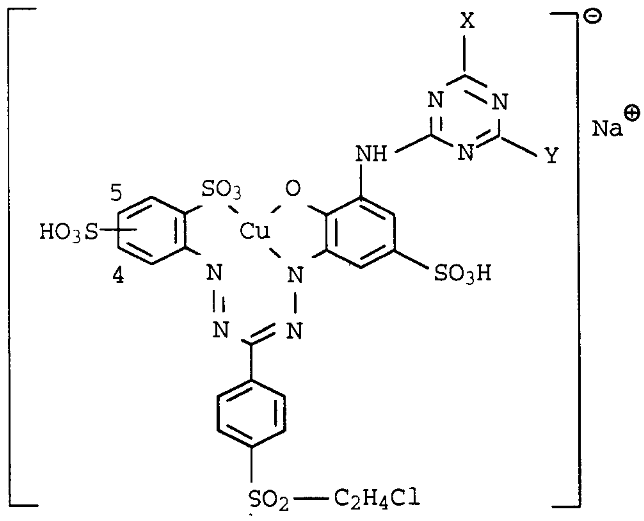

| Bsp.-Nr. | Position von $SO_3H$ | X | Y |
|---|---|---|---|
| 58 | 4 | Cl | 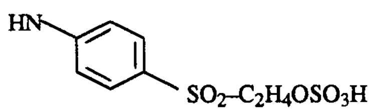 |
| 59 | 5 | Cl | 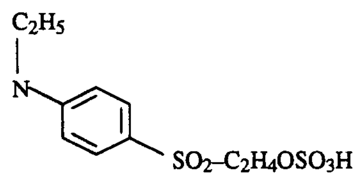 |
| 60 | 4 | F | 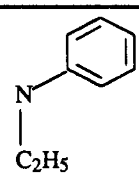 |
| 61 | 5 | Cl | 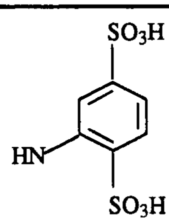 |
| 62 | 4 | Cl | $HN-C_2H_4-SO_2-C_2H_4Cl$ |
| 63 | 4 | F | $HN-C_2H_4OC_2H_4-SO_2-CH=CH_2$ |

Beispiel 64

Man verfuhr analog Beispiel 7b, verwendete jedoch anstelle von Anilin-3-sulfonsäure 15,9 g des in Beispiel 7a beschriebenen Dichlortriazinylkupferformazans, und erhielt nach dem Aussalzen einen Reaktiv-

farbstoff der Formel

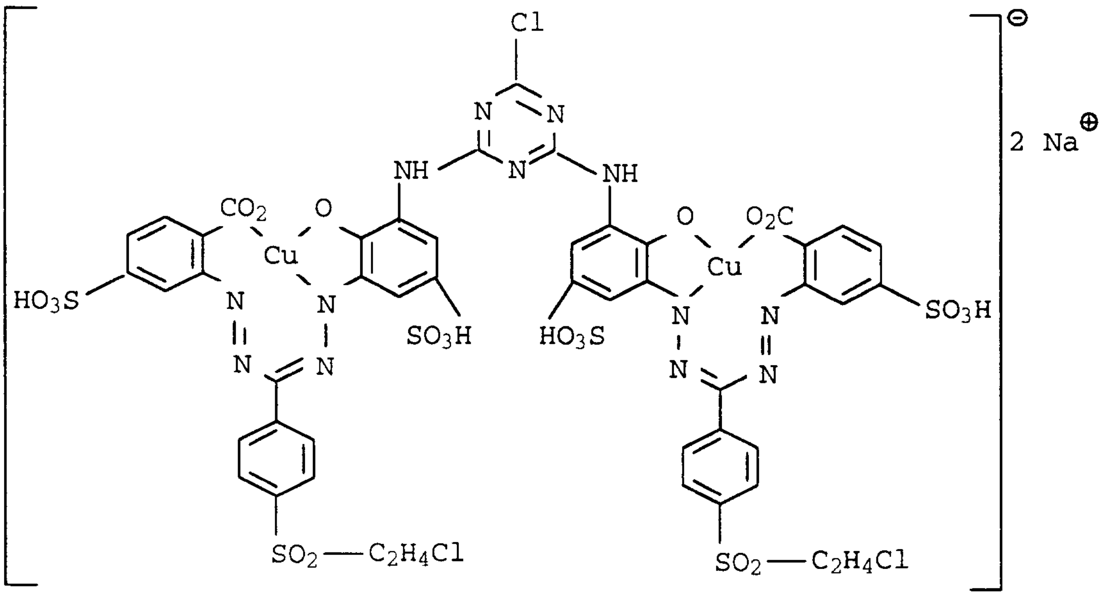

($\lambda_{max}$: 600 nm)

der Baumwolle in rotstichigem, kräftigem Blauton mit sehr guten Echtheiten färbt.

Beispiel 65

38 g des Dichlortriazins der Formel

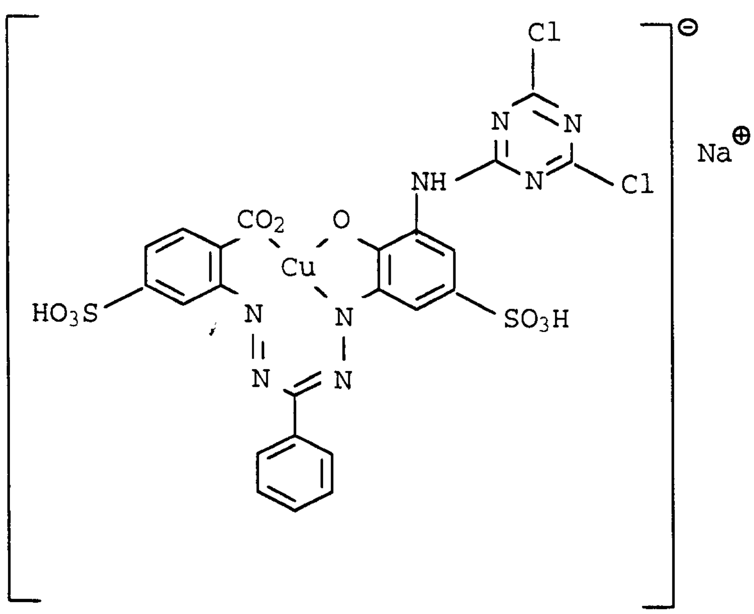

,

dessen Herstellung in Beispiel 1 der US-A-5 004 807 beschrieben ist, wurden in 500 ml Wasser bei einem pH-Wert von 6,5 und 20 bis 25 °C gelöst. Nach der Zugabe von 20,9 g des in Beispiel 1 beschriebenen Kupferformazans wurde auf 60 °C erwärmt und 3 Stunden bei dieser Temperatur nachgerührt, dabei wurde der pH-Wert mit 5 gew.-%iger wäßriger Natriumhydrogencarbonatlösung zwischen 6,0 und 6,5 gehalten. Nach dem Abkühlen auf Raumtemperatur wurde der Reaktivfarbstoff der Formel

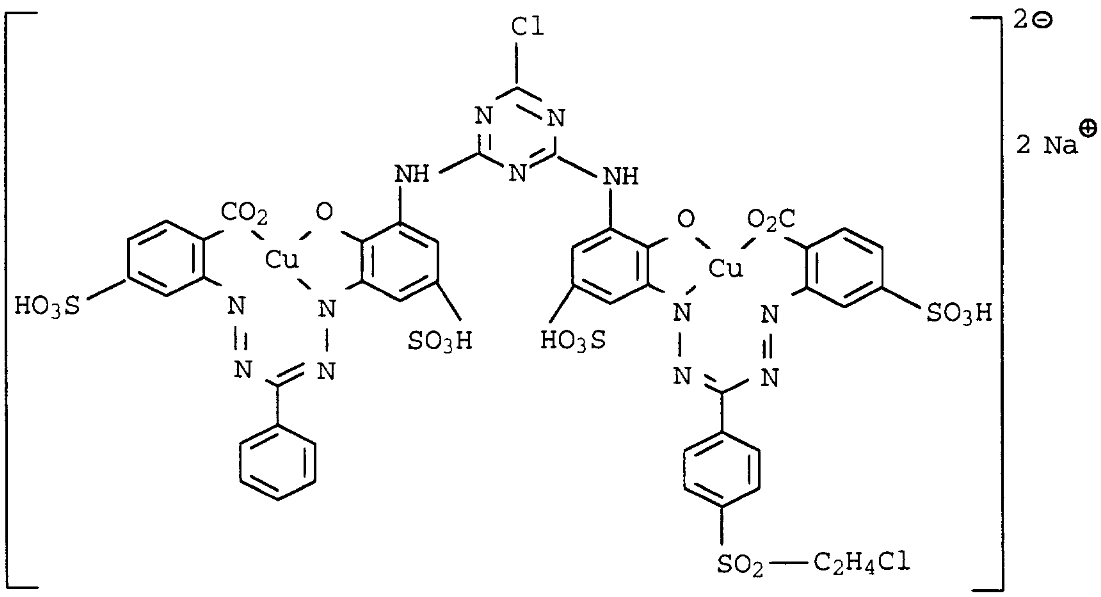

($\lambda_{max}$ = 576 nm)

erhalten, der Baumwolle in rotstichigem Blauton mit sehr guten Echtheiten färbt.

Beispiel 66

28,9 g der Kupferformazanverbindung der Formel

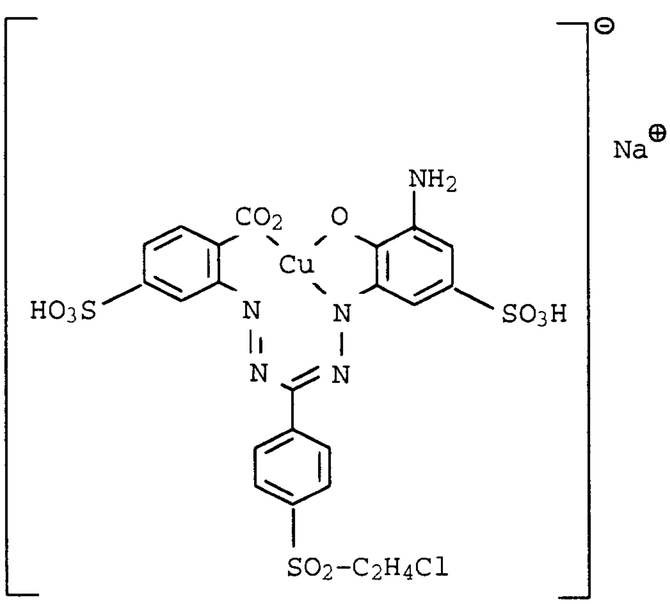

wurden in 400 ml Wasser gelöst. Man setzte 0,6 g Dinatriumhydrogenphosphat zu, kühlte auf 0 bis 5°C ab und ließ innerhalb von 5 Minuten eine Lösung von 7,4 g Cyanurchlorid in 75 ml Aceton zulaufen. Der pH-Wert wurde mit 5 gew.-%iger wäßriger Natriumhydrogencarbonatlösung bei 6,0 bis 6,5 gehalten und die Kondensation unter obigen Bedingungen zu Ende geführt (DC-Kontrolle), was ca. 1 Stunde in Anspruch nahm. Anschließend wurde eine auf einen pH-Wert von 6 eingestellte Lösung von 5,46 g 2-Methylamino-4-amino-benzolsulfonsäure in 100 ml Wasser zugegeben. Die Temperatur wurde auf 60°C erhöht, dann wurde 2 Stunden bei dieser Temperatur nachgerührt. Nach Isolierung durch Aussalzen mit 150 g Kochsalz erhielt man einen Farbstoff der Formel

($\lambda_{max}$ = 595 nm)

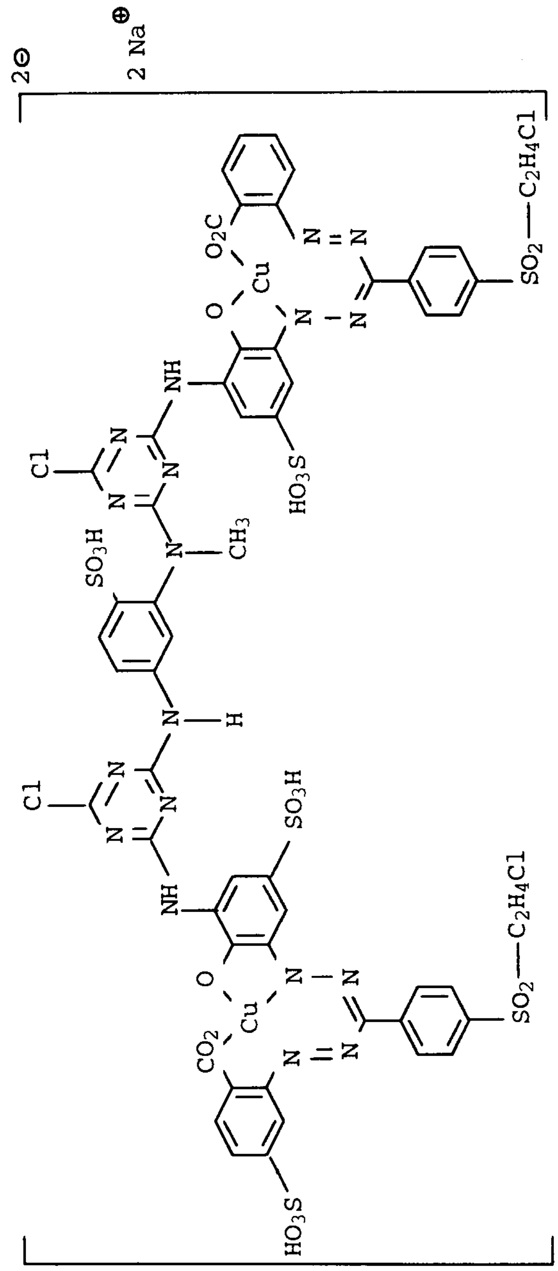

der Baumwolle in rotstichigem Blauton mit sehr guten Echtheiten färbt.

Beispiel 67

78,7 g des Aminophenols der Formel

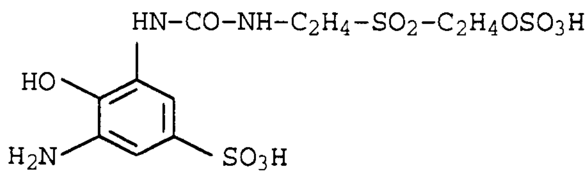

wurden in 600 ml Wasser gelöst, auf 0 bis 5 °C abgekühlt und der pH-Wert mit konz. Salzsäure auf 2 gestellt. Anschließend wurde durch Zugabe von 51 g 23 gew.-%iger wäßriger Natriumnitritlösung bei 0 bis 5 °C diazotiert.

Die resultierende Diazosuspension ließ man bei 10 bis 15 °C zu einer auf einen pH-Wert von 6,5 bis 7,0 gestellten Mischung aus 75,8 g des Hydrazons der Formel

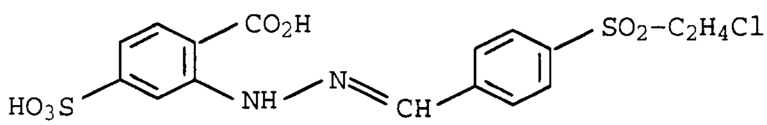

und 44,9 g Kupfersulfat-pentahydrat in 750 ml Wasser zulaufen, wobei der pH-Wert durch Einstreuen von Natriumhydrogencarbonat zwischen 6,5 und 7,0 gehalten wurde. Nach Beendigung des Zulaufs ließ man noch 12 Stunden bei 15 bis 20 °C nachrühren.

Der ausgefallene Farbstoff wurde abgesaugt und mit 20 gew.-%iger wäßriger Kochsalzlösung nachgewaschen.

Das erhaltene feuchte Nutschgut, das sich in Wasser mit blaustichig grüner Farbe löst und im UV-Spektrum charakteristische Absorptionen bei 400 nm und 633 nm zeigt, wurde zur Überführung in die stabilere Komplexform in 820 ml Wasser angerührt. Der pH-Wert wurde mit konz. Salzsäure auf 1 gestellt. Anschließend wurde 12 Stunden bei Raumtemperatur nachgerührt.

Durch Zugabe von Soda wurde auf einen pH-Wert von 6,5 bis 7,0 gestellt. Der entstandene Reaktivfarbstoff wurde durch Zusatz von 150 g Kochsalz ausgesalzen. Man erhielt einen Farbstoff der Formel

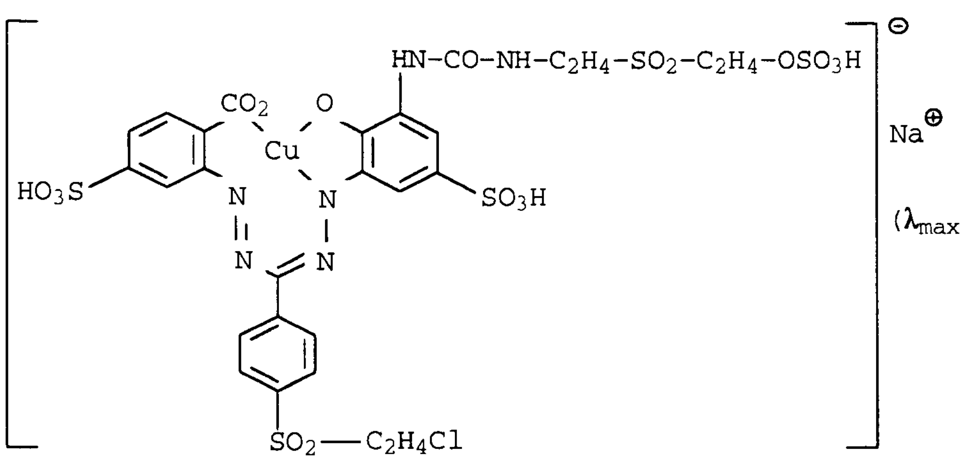

($\lambda_{max}$ = 599 nm)

der Baumwolle in kräftigem, rotstichigem Blauton mit sehr guten Echtheiten färbt.

Beispiel 68

72,6 g des in Beispiel 1 beschriebenen Kupferformazans wurden in 400 ml Wasser bei einem pH-Wert von 6 gelöst und auf 0 bis 5 °C abgekühlt. Innerhalb von 30 Minuten wurden 26,5 g des Isocyanats der Formel

$OCN\text{-}C_2H_4\text{-}SO_2\text{-}C_2H_4Cl$ ,

dessen Herstellung z.B. in Beispiel 1 der US-A-4 841 028 beschrieben ist, zugetropft, wobei der pH-Wert

bei 6,0 bis 6,2 gehalten wurde. Nach vollständigem Umsatz (DC-Kontrolle), was ca. 2 Stunden beanspruchte, wurde der gebildete Reaktivfarbstoff durch Zugabe von 150 g Kochsalz ausgefällt.

Der erhaltene Farbstoff der Formel

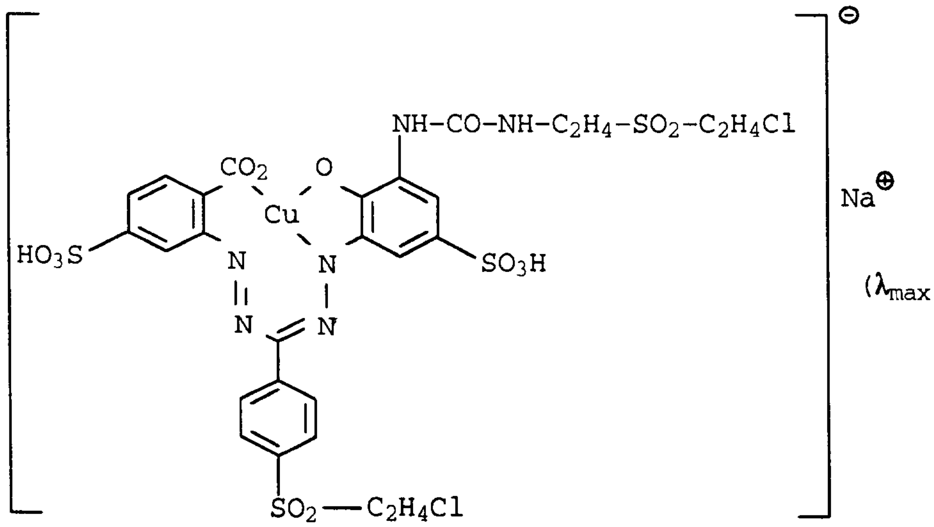

($\lambda_{max}$ = 599 nm)

färbt Baumwolle in kräftigem, rotstichigem Blauton mit sehr guten Echtheiten.

Beispiel 69

72,6 g des in Beispiel 1 beschriebenen Kupferformazans wurden in 400 ml Wasser bei einem pH-Wert von 6,5 gelöst und auf 5 bis 10 ° C abgekühlt. Innerhalb einer Stunde wurden 26,8 g des Säurechlorids der Formel

$ClCO\text{-}C_3H_6\text{-}SO_2\text{-}C_2H_4Cl$ ,

bei einem pH-Wert von 6,0 bis 6,5 und einer Temperatur von 10 ° C zugetropft. Anschließend ließ man 4 Stunden nachrühren und setzte dann 100 g Kochsalz zu. Der ausgefallene Farbstoff wurde abfiltriert und unter vermindertem Druck bei 40 ° C getrocknet.

Der erhaltene Reaktivfarbstoff der Formel

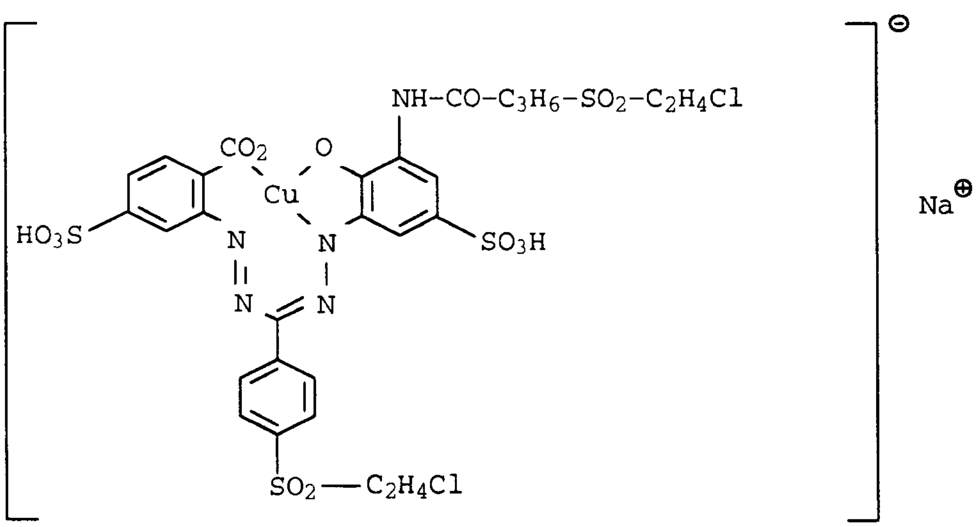

färbt Baumwolle in kräftigem, rotstichigem Blauton mit sehr guten Echtheiten.

Analog den Beispielen 67, 68 und 69 werden die in der folgenden Tabelle 4 aufgeführten Reaktivfarbstoffe erhalten.

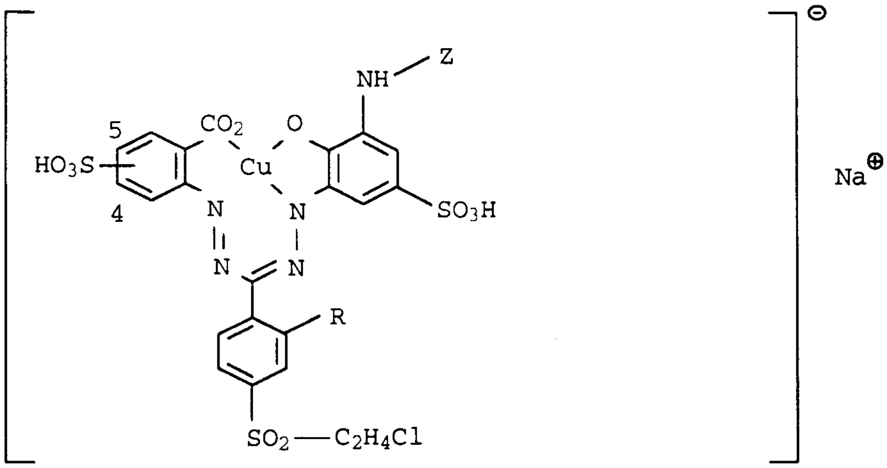

Tabelle 4

| Bsp.-Nr. | Position von $SO_3H$ | R | Z |
|---|---|---|---|
| 70 | 5 | H | $CO-NH-C_2H_4-SO_2-C_2H_4OSO_3H$ |
| 71 | 4 | Cl | $CO-NH-C_2H_4-SO_2-C_2H_4OSO_3H$ |
| 72 | 5 | H | $CO-NH-C_2H_4-SO_2-C_2H_4Cl$ |
| 73 | 4 | H | $CO-NH-C_2H_4-SO_2-C_2H_4OCOCH_3$ |
| 74 | 5 | H | $CO-NH-C_2H_4-SO_2-C_2H_4OCOCH_3$ |
| 75 | 4 | H | $CO-NH-C_3H_6-SO_2-C_2H_4OSO_3H$ |
| 76 | 4 | Cl | $CO-NH-C_3H_6-SO_2-C_2H_4OSO_3H$ |
| 77 | 5 | H | $CO-NH-C_3H_6-SO_2-C_2H_4OSO_3H$ |
| 78 | 4 | H | $CO-NCH_3-C_2H_4-SO_2-C_2H_4OSO_3H$ |
| 79 | 5 | H | $CO-NCH_3-C_2H_4-SO_2-C_2H_4OSO_3H$ |
| 80 | 4 | H | $CO-NCH_3-C_2H_4-SO_2-C_2H_4Cl$ |
| 81 | 4 | H | $CO-N(C_2H_4-SO_2-C_2H_4OSO_3H)_2$ |
| 82 | 5 | H | $CO-N(C_2H_4-SO_2-C_2H_4OSO_3H)_2$ |
| 83 | 4 | Cl | $CO-N(C_2H_4-SO_2-C_2H_4OSO_3H)_2$ |
| 84 | 4 | H | $CO-NH-C_2H_4O-C_2H_4-SO_2-C_2H_4OSO_3H$ |
| 85 | 5 | H | $CO-NH-C_2H_4O-C_2H_4-SO_2-C_2H_4OSO_3H$ |
| 86 | 4 | Cl | $CO-NH-C_2H_4O-C_2H_4-SO_2-C_2H_4OSO_3H$ |
| 87 | 4 | H | $CO-N(C_2H_4-SO_2-C_2H_4Cl)_2$ |
| 88 | 5 | H | $CO-N(C_2H_4-SO_2-C_2H_4Cl)_2$ |
| 89 | 4 | H | 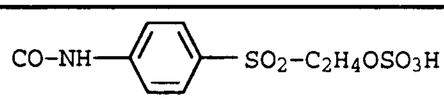 |

| | | | |
|---|---|---|---|
| 90 | 4 | H | 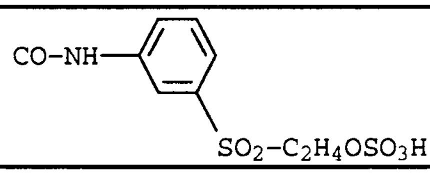 |
| 91 | 4 | H | 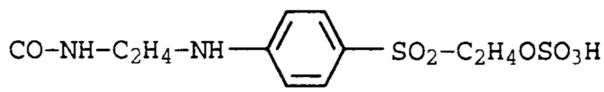 |
| 92 | 5 | H | $CO-C_3H_6-SO_2-C_2H_4Cl$ |
| 93 | 4 | Cl | $CO-C_3H_6-SO_2-C_2H_4Cl$ |
| 94 | 4 | H | 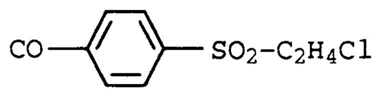 |
| 95 | 5 | H | 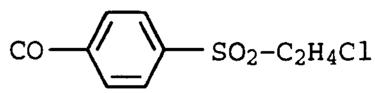 |
| 96 | 4 | H | 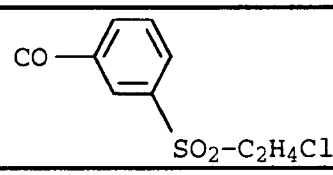 |
| 97 | 4 | Cl | 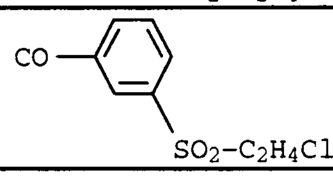 |
| 98 | 4 | H | 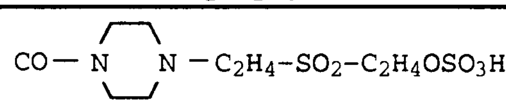 |
| 99 | 5 | H | 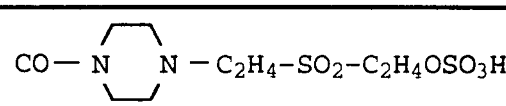 |
| 100 | 4 | H | 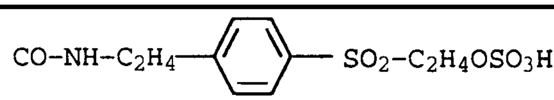 |

| | | | |
|---|---|---|---|
| 101 | 5 | H | 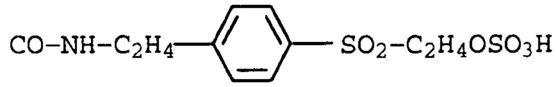 |
| 102 | 4 | H | 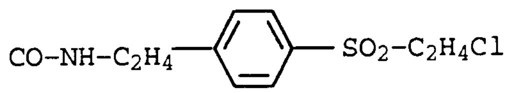 |

Beispiel 103

a) Zu 160 g Natriumhydroxid in 1,5 l Wasser wurden unter Kühlung bei 20 bis 40 °C 232 g 3-Amino-4-hydroxy-5-nitrobenzolsulfonsäure eingetragen. Die Reaktionsmischung wurde auf 0 bis 5 °C abgekühlt, dann wurden innerhalb von 8 Stunden 182 g Phosgen eingeleitet, wobei der pH-Wert mit 150 g 50 gew.-%iger Natronlauge ≧ 10 gehalten wurde. Es wurde eine Stunde nachgerührt und anschließend überschüssiges Phosgen mit Stickstoff ausgeblasen. Der pH-Wert wurde mit 200 g konz. Salzsäure auf 7 gestellt. Zur entstandenen 7-Nitrobenzoxazol-2-on-5-sulfonsäure (Natriumsalz) wurden 133 g 2'-Aminoethyl-2-hydroxyethylsulfid gegeben und der pH-Wert mit 164 g konz. Salzsäure auf 7 gestellt. Es wurde auf 60 °C erwärmt und 6,5 Stunden bei dieser Temperatur gerührt. Nach vollständigem Umsatz (DC-Kontrolle) wurde mit konz. Salzsäure auf einen pH-Wert von 5 gestellt, 0,50 g Wolframsäure zugegeben und anschließend innerhalb von 2 Stunden 340 g 30 gew.-%iges Wasserstoffperoxid zugetropft. Es wurde weitere 2 Stunden bei 60 °C bis zum vollständigen Umsatz (DC-Kontrolle) nachgerührt. Nach dem Abkühlen auf 0 bis 5 °C wurde mit 150 g konz. Salzsäure ein pH-Wert von 0,5 bis 1,0 eingestellt und das ausgefallene Produkt abgesaugt. Nach dem Trocknen bei 70 bis 80 °C unter vermindertem Druck erhielt man 377 g einer Verbindung der Formel

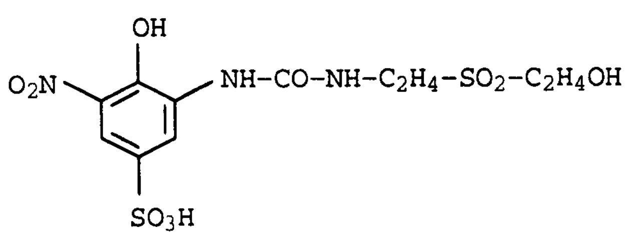

$^1$H-NMR [$D^6$-DMSO] : $\delta$ = 3,3 ($CH_2$), 3,4 ($CH_2$), 3,6 ($CH_2$), 3,8 ($CH_2$), 5,2 (OH), 7,3 (NH), 7,7, 8,6 (Aromaten-H), 11,0 (NH) ppm

b) 306 g der unter a) erhaltenen Nitroverbindung wurden in 3 l Wasser gelöst. Man gab 5 g Palladiumkatalysator (10 gew.-%ig auf Kohle) zu und hydrierte bei 35 bis 40 °C. Nach beendeter Wasserstoffaufnahme wurde vom Katalysator abfiltriert und die Mutterlauge eingedampft. Es verblieben 280 g der Verbindung der Formel

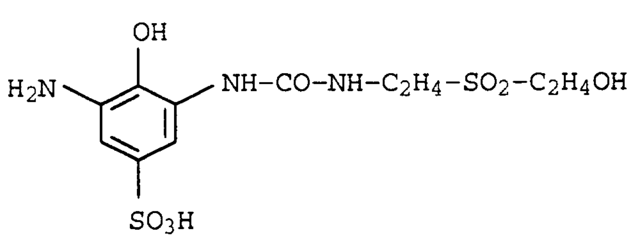

$^1$H-NMR [$D^6$-DMSO] : $\delta$ = 3,30, 3,35, 3,55, 3,80 (jeweils $CH_2$), 4,00 (OH), 7,10 (NH), 7,35, 7,50

(Aromaten-H), 9,20 (OH) 11,5 (NH)

Beispiel 104

280 g des in Beispiel 103b erhaltenen Aminophenols wurden unter Eiskühlung bei 10 bis 15 °C in 1120 g Chlorsulfonsäure eingetragen und ca. 12 Stunden bei Raumtemperatur gerührt. Nach vollständigem Umsatz (DC-Kontrolle) wurde auf 4 l Eis gefällt, das ausgefallene Produkt abgesaugt und mit Wasser neutralgewaschen. Nach dem Trocknen unter vermindertem Druck bei 30 bis 40 °C verblieben 302 g der Verbindungder Formel

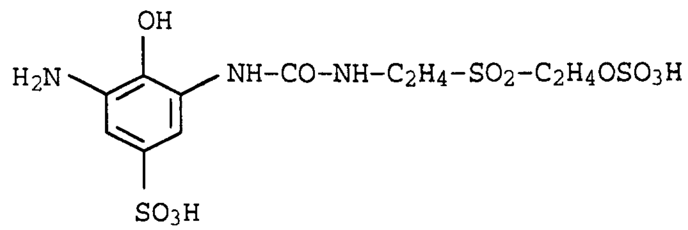

die ohne weitere Reinigung zu Farbstoffsynthesen wie beispielsweise in Beispiel 67 beschrieben, verwendet werden kann.

Beispiel 105

a) Man verfuhr analog Beispiel 103a, verwendete jedoch anstelle von 3-Amino-4-hydroxy-5-nitrobenzolsulfonsäure 232 g der isomeren 2-Hydroxy-3-amino-5-nitrobenzolsulfonsäure. Man erhielt nach dem Trocknen bei 70 bis 80 °C unter vermindertem Druck 365 g einer Verbindung der Formel

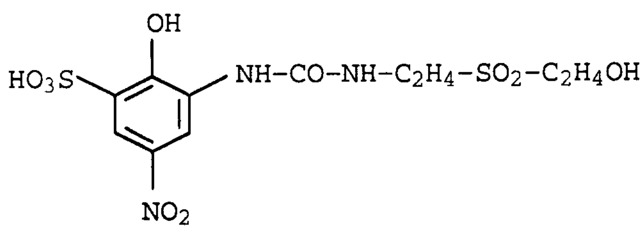

b) Man reduzierte die unter a) erhaltene Nitroverbindung gemäß Beispiel 103b und veresterte 280 g der Aminoverbindung mit Chlorsulfonsäure analog Beispiel 104. Man erhielt nach dem Trocknen bei 30 bis 40 °C 295 g der Verbindung der Formel

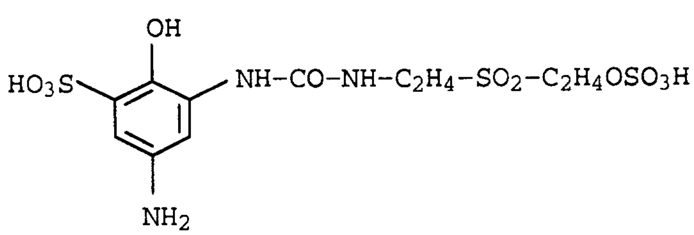

Analog Beispiel 103 oder 104 werden die in der folgenden Tabelle 5 aufgeführten Aminophenole erhalten

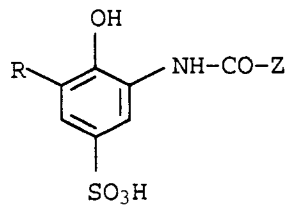

| Bsp. Nr. | R | Z |
|---|---|---|
| 106 | $NO_2$ | $NHC_3H_6SO_2C_2H_4OH$ |
| 107 | $NH_2$ | $NHC_3H_6SO_2C_2H_4OH$ |
| 108 | $NH_2$ | $NHC_3H_6SO_2C_2H_4OSO_3H$ |
| 109 | $NO_2$ | $N(CH_3)C_2H_4SO_2C_2H_4OH$ |
| 110 | $NH_2$ | $N(CH_3)C_2H_4SO_2C_2H_4OH$ |
| 111 | $NH_2$ | $N(CH_3)C_2H_4SO_2C_2H_4OSO_3H$ |
| 112 | $NO_2$ | $NHC_2H_4OC_2H_4SO_2C_2H_4OH$ |
| 113 | $NH_2$ | $NCH_2H_4OC_2H_4SO_2C_2H_4OH$ |
| 114 | $NH_2$ | $NHC_2H_4OC_2H_4SO_2C_2H_4OSO_3H$ |
| 115 | $NO_2$ | 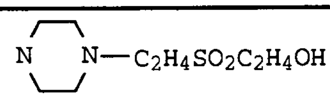 |
| 116 | $NH_2$ | 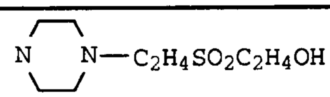 |
| 117 | $NH_2$ | 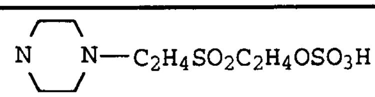 |
| 118 | $NO_2$ | $N(C_2H_4SO_2C_2H_4OH)_2$ |
| 119 | $NH_2$ | $N(C_2H_4SO_2C_2H_4OH)_2$ |
| 120 | $NH_2$ | $N(C_2H_4SO_2C_2H_4OSO_3H)_2$ |
| 121 | $NO_2$ | 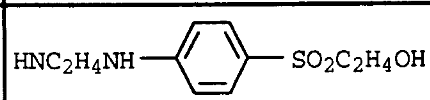 |
| 122 | $NH_2$ | 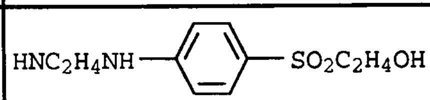 |
| 123 | $NH_2$ | 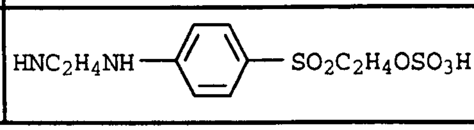 |

| Bsp. Nr. | R | Z |
|---|---|---|
| 124 | $NO_2$ | 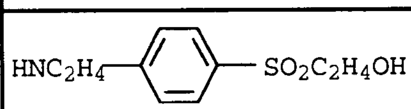 |
| 125 | $NH_2$ | 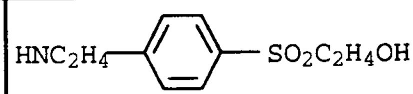 |
| 126 | $NH_2$ | 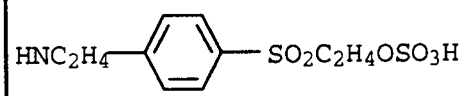 |

## Patentansprüche

**1.** Formazanfarbstoffe der Formel I

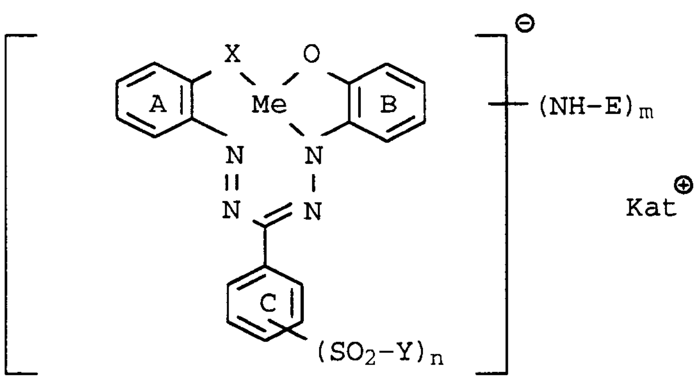

(I),

in der

| | |
|---|---|
| m und n | unabhängig voneinander jeweils 1 oder 2, |
| $Kat^{\oplus}$ | das Äquivalent eines Kations, |
| Me | Kupfer oder Nickel, |
| X | Sauerstoff oder einen Rest der Formel CO-O oder $SO_2$-O, |
| Y | Vinyl oder einen Rest der Formel $C_2H_4$-Q, worin Q für eine unter alkalischen Reaktionsbedingungen abspaltbare Gruppe steht, und |
| E | einen heterocyclischen Ankerrest oder einen Ankerrest der Formel L-W, worin L für ein Brückenglied und W für einen Ankerrest aus der aliphatischen Reihe stehen, bedeuten und |

die Ringe A und B gegebenenfalls substituiert sind und benzoanelliert sein können und der Ring C gegebenenfalls substituiert ist.

**2.** Formazanfarbstoffe nach Anspruch 1, die der Formel Ia

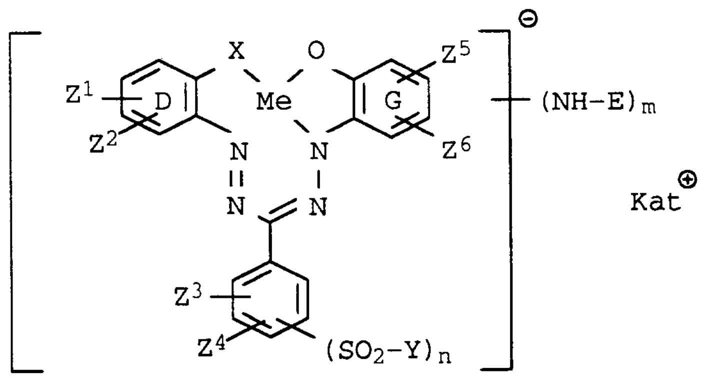

(Ia)

entsprechen, in der

- $Z^1$ Wasserstoff oder Hydroxysulfonyl,
- $Z^2$ Wasserstoff, $C_1$-$C_4$-Alkanoylamino, Halogen oder Nitro,
- $Z^3$ und $Z^4$ unabhängig voneinander jeweils Wasserstoff, Halogen, $C_1$-$C_4$-Alkyl, $C_1$-$C_4$-Alkoxy, $C_1$-$C_4$-Alkylsulfonyl, Nitro oder Hydroxysulfonyl und
- $Z^5$ und $Z^6$ unabhängig voneinander jeweils Wasserstoff, Hydroxysulfonyl, Halogen, Nitro, Carboxyl, Ureido, gegebenenfalls substituiertes $C_1$-$C_4$-Mono- oder Dialkylureido, gegebenenfalls substituiertes Phenylureido, gegebenenfalls substituiertes Phenyl, gegebenenfalls substituiertes $C_1$-$C_4$-Alkanoylamino, gegebenenfalls substituiertes Benzoylamino, $C_1$-$C_4$-Alkoxycarbonylamino,gegebenenfalls substituiertes Mono- oder Dialkylcarbamoyl oder gegebenenfalls substituiertes Mono- oder Dialkylsulfamoyl bedeuten,

die Ringe D und G benzoanelliert sein können und m, n, $Kat^{\oplus}$, Me, E, X und Y jeweils die in Anspruch 1 genannte Bedeutung besitzen.

3. Formazanfarbstoffe nach Anspruch 1, dadurch gekennzeichnet, daß Me Kupfer bedeutet.

4. Formazanfarbstoffe nach Anspruch 1, dadurch gekennzeichnet, daß m und n jeweils 1 bedeuten.

5. Formazanfarbstoffe nach Anspruch 1, dadurch gekennzeichnet, daß Y Vinyl oder einen Rest der Formel $C_2H_4$-Q bedeutet, worin Q für Chlor, Sulfato oder Thiosulfato steht.

6. Formazanfarbstoffe nach Anspruch 1, dadurch gekennzeichnet, daß X den Rest der Formel CO-O bedeutet.

7. Verwendung der Formazanfarbstoffe gemäß Anspruch 1 zum Färben oder Bedrucken von Hydroxygruppen oder Stickstoffatome aufweisenden organischen Substraten.

8. Aminophenole der Formel II

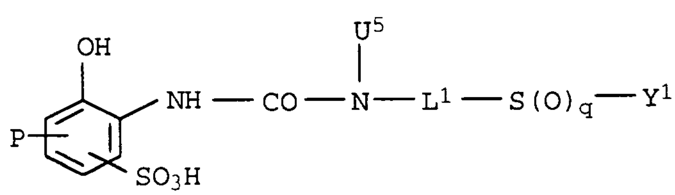

(II),

in der

- q 0 oder 2,
- $Y^1$ Vinyl oder einen Rest der Formel $C_2H_4$-$Q^1$, worin $Q^1$ für eine unter alkalischen Reaktionsbedingungen abspaltbare Gruppe oder Hydroxy steht,
- $L^1$ $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 bis 2 Sauerstoffatome in Etherfunktion oder Imino- oder $C_1$-$C_4$-Alkyliminogruppen unterbrochen sein kann,
- $U^5$ Wasserstoff, $C_1$-$C_6$-Alkyl, das gegebenenfalls durch Hydroxy, $C_1$-$C_4$-Alkoxy, Halogen, Cyano,

Hydroxysulfonyl oder einen Rest der Formel $-SO_2-Y^1$, wobei $Y^1$ die obengenannte Bedeutung besitzt, substituiert ist, oder gegebenenfalls substituiertes Phenyl und
P Nitro oder Amino bedeuten.

**9.** Verfahren zur Herstellung von Aminophenolen der Formel II

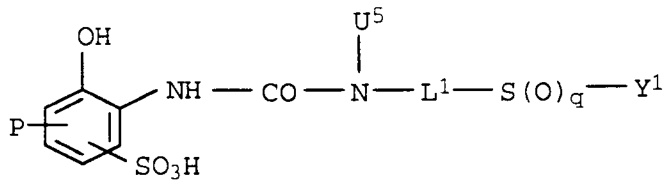

(II),

in der
q 0 oder 2,
$Y^1$ Vinyl oder einen Rest der Formel $C_2H_4-Q^1$, worin $Q^1$ für eine unter alkalischen Reaktionsbedingungen abspaltbare Gruppe oder Hydroxy steht,
$L^1$ $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 bis 2 Sauerstoffatome in Etherfunktion oder Imino- oder $C_1$-$C_4$-Alkyliminogruppen unterbrochen sein kann,
$U^5$ Wasserstoff, $C_1$-$C_6$-Alkyl, das gegebenenfalls durch Hydroxy, $C_1$-$C_4$-Alkoxy, Halogen, Cyano, Hydroxysulfonyl oder einen Rest der Formel $-SO_2-Y^1$, wobei $Y^1$ die obengenannte Bedeutung besitzt, substituiert ist, oder gegebenenfalls substituiertes Phenyl und
P Nitro oder Amino bedeuten,
dadurch gekennzeichnet, daß man ein Benzoxazolon der Formel III

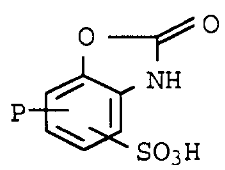

(III),

in der P die obengenannte Bedeutung besitzt, mit einem Amin der Formel IV

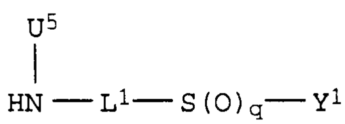

(IV),

in der q, $U^5$, $L^1$ und $Y^1$ jeweils die obengenannte Bedeutung besitzen, umsetzt.